# EUROPEAN PATENT APPLICATION

(11) **EP 4 053 096 A1**
(43) Date of publication of application: **07.09.2022**
(21) Application number: 21305263.2
(22) Date of filing: 05.03.2021
(51) Int. Cl.: C07C 46/02, C07C 50/14

(54) **PHOTOREDOX RADICAL BENZYLATION PROCESS**

(71) Applicant: Centre national de la recherche scientifique, 75016 Paris (FR); Université de Strasbourg, 67000 Strasbourg (FR); Université de Haute Alsace, 68093 Mulhouse Cedex (FR)
(72) Inventor: DAVIOUD-CHARVET, Elisabeth, 67000 Strasbourg (FR); DONZEL, Maxime, 67000 Strasbourg (FR); ELHABIRI, Mourad, 67200 Strasbourg (FR)
(74) Representative: Lavoix

(57) **Abstract**

The present invention relates to a method for the preparation of a compound having the formula (I): 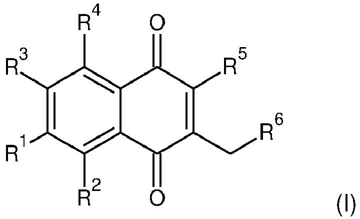 wherein:
• R¹, R², R³, and R⁴ are in particular H or halogen,
• R⁵ is in particular (C₁-C₆)alkyl,
• R⁶ is a group of formula -CH=CH₂ or a group having the below formula (II): 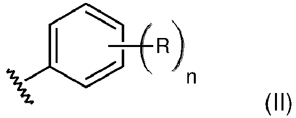 said method comprising a reacting step carried out under light irradiation in the presence of an iron catalyst, a heteroatomic base, and a HAT agent.

## Description

The present invention concerns a photoredox radical benzylation process, in particular implemented to convert quinones in one step to a large library of diversely substituted compounds.

Recently, benzylated quinones have shown an increasing interest in medicinal chemistry, especially in the field of the neglected tropical parasitic diseases such as malaria. Plasmodione, for instance, has shown interesting anti-parasitic activities against *Plasmodium falciparum in vitro* and *Plasmodium berghei* in the mouse model (Müller, T.; Johann, L.; Jannack, B.; Brückner, M.; Lanfranchi, D. A.; Bauer, H.; Sanchez, C.; Yardley, V.; Deregnaucourt, C.; Schrével, J.; Lanzer, M.; Schirmer, R. H.; Davioud-Charvet, E. Glutathione reductase-catalyzed cascade of redox reactions to bioactivate potent antimalarial 1,4-naphthoquinones - A new strategy to combat malarial parasites. J. Am. Chem. Soc. 2011, 133, 11557-71. doi: 10.1021/ja201729z) This 2-methylnapthoquinone is benzylated on its 3-position. This has led to the development of new synthetic methodologies to introduce structural and functional diversity for reaching optimized and more active compounds. The original synthetic route involved a Kochi-Anderson radical decarboxylation of phenyl acetic acids to generate the benzyl radicals (Anderson, J. M.; Kochi, J. K. Silver (I)-catalyzed oxidative decarboxylation of acids by peroxydisulfate. the role of silver (II). J. Am. Chem. Soc. 1970, 92, 1651-1659. Doi: 10.1021/ja00709a039) but was suffering from the need of silver salts and the use of phenylacetic acids as starting materials. This pioneer work to benzylate quinones was described by Jacobsen & Torssell ((a) Jacobsen N.; Torssell K. Synthesis of Naturally Occurring Quinones. Alkylation with the Silver lon-Peroxydisulphate-Carboxylic Acid System. Acta Chem. Scand. 1973, 27, 3211-3216. Doi: 10.3891/acta.chem.scand.27-3211. (b) Goldman, J.; Jacobsen, N.; Torssell, K. Syntheses in the Camphor Series. Alkylation of Quinones with Cycloalkyl Radicals. Attempted Syntheses of Lagopodin A and Desoxyhelicobasidin. Acta Chem. Scand. 1974, 28b, 492-500. Doi: 10.3891/acta.chem.scand.28b-0492). More recently, Lee's group developed an elegant silver free alkylation of quinones in mild conditions in the presence of (NH₄)₂S₂O₈ in DMSO and water, although this method is efficient on quinones and a great variety of heteroarenes, it suffers of the great amount (10 mmol) of starting acid mandatory, making expensive to use highly substituted starting acids (Sutherland, D. R.; Veguillas, M.; Oates, C. L.; Lee, A.-L. Metal-, Photocatalyst-, and Light-Free, Late-Stage C-H Alkylation of Heteroarenes and 1,4-Quinones Using Carboxylic Acids. Org. Lett. 2018, 20, 6863-6867. doi: 10.1021/acs.orglett.8b02988). Witness to the growing interest in benzylated quinones, the synthesis of such benzylated naphthoquinones, including plasmodione, was achieved several times last years from substituted toluenes by free-radical generation via hydrogen abstraction using a couple selectfluor/Ag(I) (Galloway J. D., Mai D. N., Baxter R. D. Radical Benzylation of Quinones via C-H Abstraction. J. Org. Chem., 2019, 84, 12131-12137. doi: 10.1021/acs.joc.9b01004) or peroxides ((a) Zhou, S.-L., Guo, L.-N. and Duan, X.-H. Copper-Catalyzed Regioselective Cross-Dehydrogenative Coupling of Coumarins with Benzylic Csp3-H Bonds. Eur. J. Org. Chem. 2014, 8094-8100. doi: 10.1002/ejoc.201403068. (b) Dong Y., Yang J., He S., Shi Z.-C., Wang Y., Zhang X.-M., Wang J.-Y. Metal-free oxidative cross-dehydrogenative coupling of quinones with benzylic C(sp3)-H bonds. RSC Adv. 2019, 9, 27588-27592. doi: 10.1039/c9ra05678e). Finally, Li & Shen in 2020 showed that benzyl radicals can be generated at high temperature in the presence of Iron(II) and a base to react afterwards with quinones or coumarins (Li D., Shen X. Iron-catalyzed regioselective alkylation of 1,4-quinones and coumarins with functionalized alkyl bromides. Org Biomol Chem. 2020, 18, 750-754. doi: 10.1039/c9ob02289a).

There is thus a need for a process for the preparation of 3-benzylmenadiones and derivatives thereof able to overcome the limitations of the silver-catalyzed decarboxylative benzylation.

The aim of the present invention is thus to provide an efficient method for generating benzyl radicals from cheap, diversely substituted, and easily available benzyl precursors.

Another aim of the present invention is to provide a photoredox method that is implemented in a single step, and that gives a large library of diversely substituted compounds.

Therefore, the present invention relates to a method for the preparation of a compound having the formula (I): 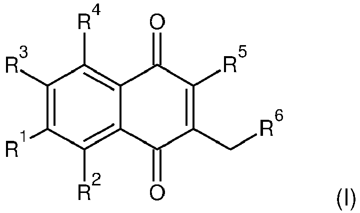 wherein:
- R¹, R², R³, and R⁴ are independently from each other selected from the group consisting of:
   □ H,
   □ halogen,
   □ OH,
   □ (C₁-C₆)alkyl,
   □ phosphate di(C₁-C₆)alkyl ester,
   □ halo(C₁-C₆)alkyl,
   □ halo(C₁-C₆)alkoxy, and
   □ (C₁-C₆)alkoxy,
- R⁵ is selected from the group consisting of:
   □ (C₁-C₆)alkyl,
   □ OH,
   □ halogen,
   □ (C₆-C₁₀)aryl, such as phenyl,
   □ (C₆-C₁₀)aryloxy, such as phenyloxy,
   □ (C₆-C₁₀)aryl(C₁-C₆)alkyl, such as benzyl, and
   □ -O-C(=O)-(C₁-C₆)alkyl, such as OAc,
- R⁶ is a group of formula -CH=CH₂ or a group having the below formula (II): 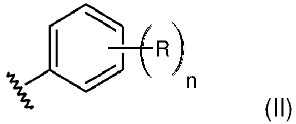 wherein:
   □ n is 0 or an integer varying from 1 to 5; and
   □ R, identical or different, is selected from the group consisting of:
      - halo(C₁-C₆)alkyl,
      - (C₁-C₆)alkyl,
      - CN,
      - NO₂,
      - halogen,
      - -C(=O)-(C₁-C₆)alkyl,
      - halo(C₁-C₆)alkoxy,
      - (C₁-C₆)alkoxy,
   or two adjacent R groups may form together with the carbon atoms carrying them a (C₆-C₁₀)aryl group or a heterocycloalkyl group;
   said method comprising a step of reacting a compound having the following formula (III): 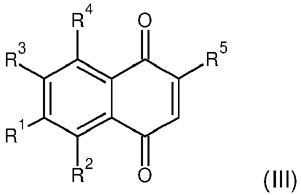
   R¹, R², R³, R⁴, and R⁵ being as defined above in formula (I);
   with a compound having the following formula (IV):

      X-CH₂-R⁶ (IV)

      R⁶ being as defined above in formula (I), and
      X being a halogen atom, preferably Br;
      said reacting step being carried out under light irradiation in the presence of an iron catalyst, a heteroatomic base, and a HAT agent.

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 6 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heterocycloalkyl group" means: a 4- to 10-membered, saturated or partially unsaturated, monocyclic or bicyclic group comprising from one to three heteroatoms selected from O, S or N; the heterocycloalkyl group may be attached to the rest of the molecule via a carbon atom or via a heteroatom; the term bicyclic heterocycloalkyl includes fused bicycles and spiro-type rings.

By way of saturated heterocycloalkyl comprising from 5 to 6 atoms, mention may be made of oxetanyl, tetrahydrofuranyl, dioxolanyl, pyrrolidinyl, azepinyl, oxazepinyl, pyrazolidinyl, imidazolidinyl, tetrahydrothiophenyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

Among the heterocycloalkyls, mention may also be made, by way of examples, of bicyclic groups such as (8aR)-hexahydropyrrolo[1,2-a]pyrazin-2(1H)-yl, octahydroindozilinyl, diazepanyl, dihydroimidazopyrazinyl and diazabicycloheptanyl groups, or else diazaspiro rings such as 1,7-diazaspiro[4.4]non-7-yl or 1-ethyl-1,7-diazaspiro[4.4]non-7-yl.

When the heterocycloalkyl is substituted, the substitution(s) may be on one (or more) carbon atom(s) and/or on the heteroatom(s). When the heterocycloalkyl comprises several substituents, they may be borne by one and the same atom or different atoms.

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

The term "phosphate di(C₁-C₆)alkyl ester" refers to a group of formula -O(P=O)(OAlk)₂, Alk being a (C₁-C₆)alkyl group as defined above. By way of example, mention may be made of phosphate diethyl ester of formula -O(P=O)(OEt)₂.

The term "halogen" means: a fluorine, a chlorine, a bromine or an iodine.

The term "alkoxy group" means: an -O-alkyl radical where the alkyl group is as previously defined. By way of examples, mention may be made of -O-(C₁-C₄)alkyl groups, and in particular the -O-methyl group, the -O-ethyl group as -O-C₃alkyl group, the -O-propyl group, the -O-isopropyl group, and as -O-C₄alkyl group, the -O-butyl, -O-isobutyl or -O-tert-butyl group.

The term "haloalkyl group" means: an alkyl group as defined above, in which one or more of the hydrogen atoms is (are) replaced with a halogen atom. By way of example, mention may be made of fluoroalkyls, in particular CF₃ or CHF₂.

The term "haloalkoxy group" means: an -O-haloalkyl group, the haloalkyl group being as defined above. By way of example, mention may be made of fluoroalkyls, in particular OCF₃ or OCHF₂.

The abovementioned "alkyl", "aryl" and "heterocycloalkyl" radicals can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "heteroalkyl group" means: an alkyl group as defined above, in which one or more of the carbon atoms is (are) replaced with a heteroatom, such as O or N.

The term "carboxyl" means: a COOH group.

The term "oxo" means: "=O".

The method of the present invention is based on the use of a compound of formula (IV) being CH₂=CH-CH₂-X, preferably CH₂=CH-CH₂-Br, or being a compound of formula (II-1):

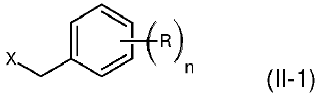

X, n, and R being as defined above in formula (II).

Preferably, the compound of formula (IV) has the following formula (II-2): 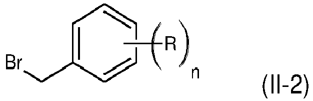 n and R being as defined above in formula (II).

According to an embodiment, a family of compounds prepared by the method according to the present invention consists of compounds having the following formula (I'): 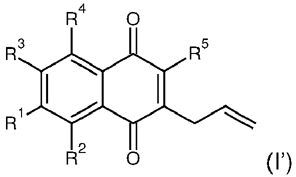 wherein R¹, R², R³, R⁴, and R⁵ are as defined above in formula (I).

The compounds of formula (I') are compounds of formula (I) wherein R⁶ is a group of formula -CH=CH₂.

A compound of formula (I') is for example the following compound:

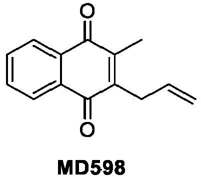

According to an embodiment, a family of compounds prepared by the method according to the present invention consists of compounds having the following formula (I-1): 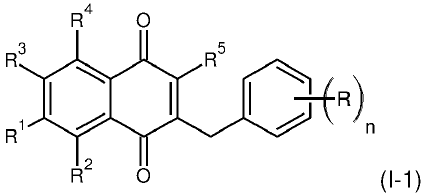 wherein R¹, R², R³, R⁴, R⁵, R, and n are as defined above in formula (I).

According to an embodiment, in formula (I) or (I-1) as defined above, n is 0, 1, 2, 3 or 5.

According to an embodiment, a family of compounds prepared by the method according to the present invention consists of compounds having the following formula (I-1-1): 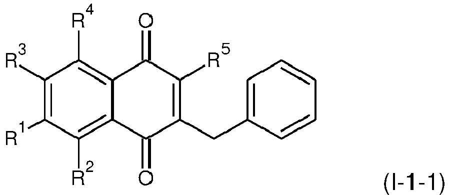 wherein R¹, R², R³, R⁴, and R⁵ are as defined above in formula (I).

As compounds of formula (1-1-1), the followings may be mentioned:

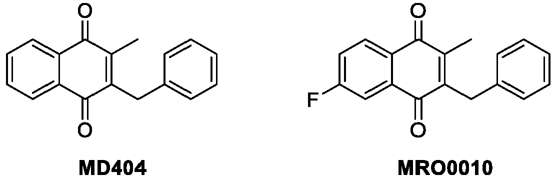

According to an embodiment, a family of compounds prepared by the method according to the present invention consists of compounds having the following formula (I-2): 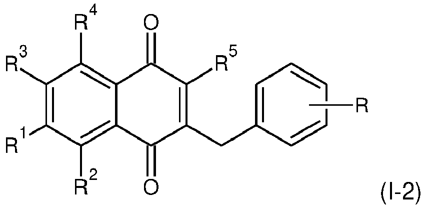 wherein R¹, R², R³, R⁴, R⁵, and R are as defined above in formula (I).

According to an embodiment, a family of compounds prepared by the method according to the present invention consists of compounds having the following formula (I-3): 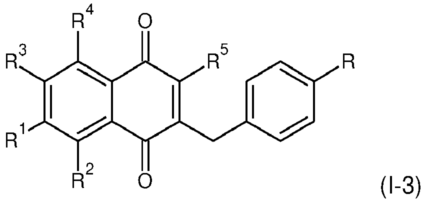 wherein R¹, R², R³, R⁴, R⁵, and R are as defined above in formula (I).

As compounds of formula (I-3), the followings may be mentioned:

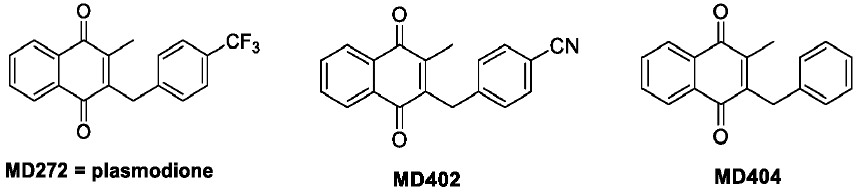

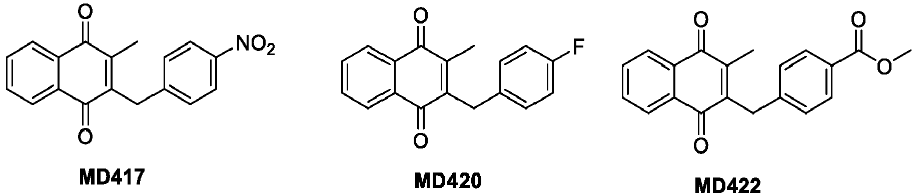

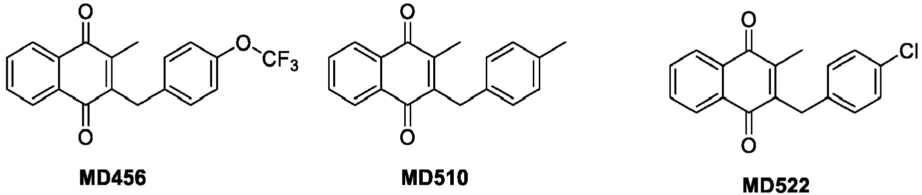

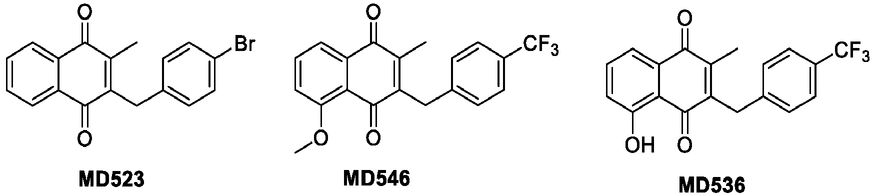

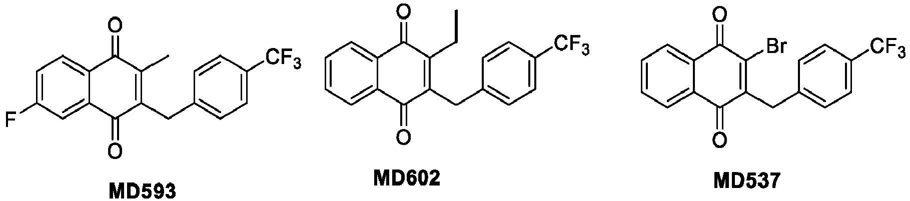

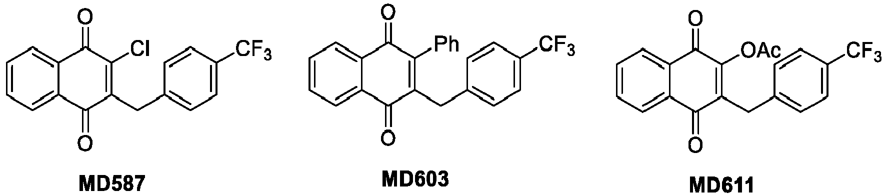

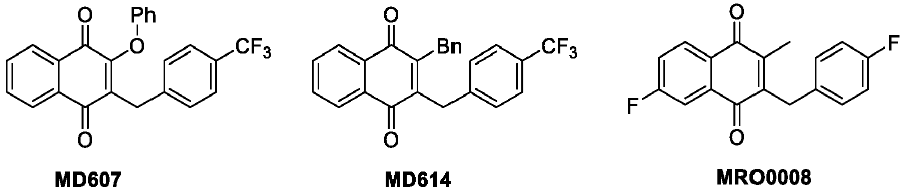

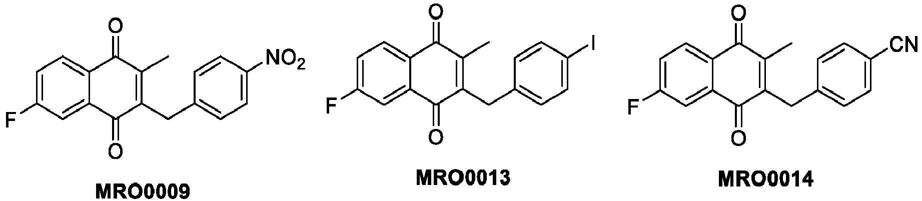

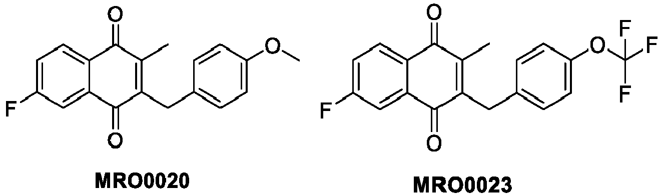

According to an embodiment, a family of compounds prepared by the method according to the present invention consists of compounds having the following formula (I-4): 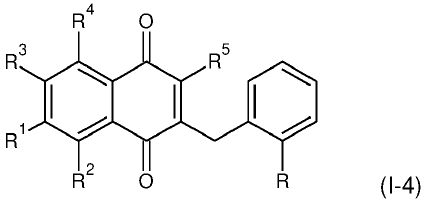 wherein R¹, R², R³, R⁴, R⁵, and R are as defined above in formula (I).

As compounds of formula (I-4), the followings may be mentioned:

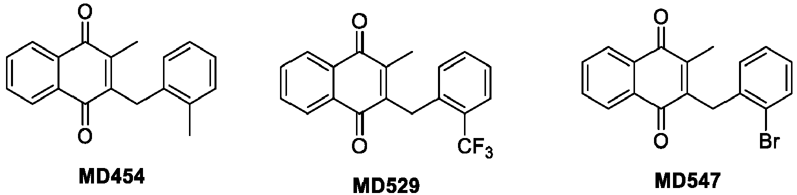

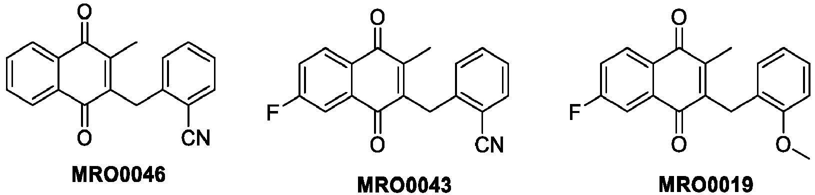

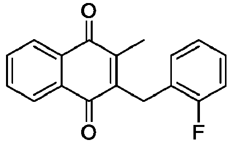

According to an embodiment, a family of compounds prepared by the method according to the present invention consists of compounds having the following formula (I-5): 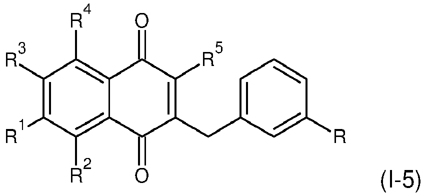 wherein R¹, R², R³, R⁴, R⁵, and R are as defined above in formula (I).

As compounds of formula (I-5), the followings may be mentioned:

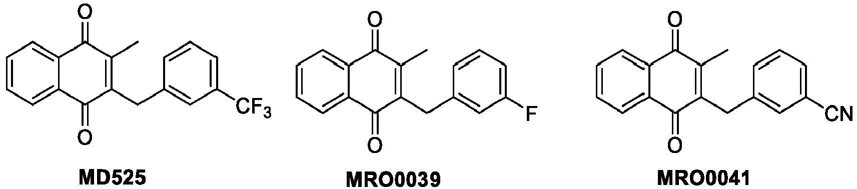

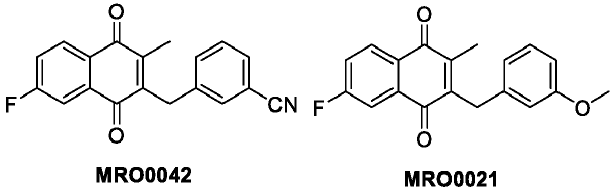

Preferably, in formula (I-3), R is selected from the group consisting of: halo(C₁-C₆)alkyl, in particular CF₃, CN, NO₂, halogen such as I, Cl, Br or F, (C₁-C₆)alkyl such as methyl, -C(=O)-(C₁-C₆)alkyl such as COOMe, halo(C₁-C₆)alkoxy, such as OCF₃, and (C₁-C₆)alkoxy, such as OMe.

Preferably, in formula (I-4), R is selected from the group consisting of: CN, (C₁-C₆)alkyl such as methyl, halo(C₁-C₆)alkyl, in particular CF₃, halogen such as Br or F, and (C₁-C₆)alkoxy,such as OMe.

Preferably, in formula (I-5), R is selected from the group consisting of: CN, halogen such as F, and (C₁-C₆)alkoxy, such as OMe.

According to an embodiment, a family of compounds prepared by the method according to the present invention consists of compounds having the following formula (I-6): 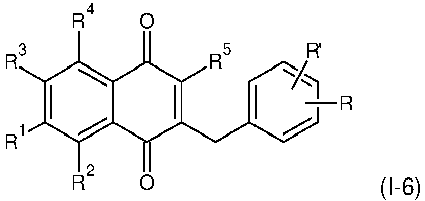
wherein R¹, R², R³, R⁴, R⁵, and R are as defined above in formula (I),
and wherein R', identical or different, has the same definition as R.

Preferably, in formula (I-6), R and R', identical or different, are selected from the group consisting of: NO₂, (C₁-C₆)alkyl such as methyl, halogen such as F, Cl, Br or I, halo(C₁-C₆)alkyl, in particular CF₃, and (C₁-C₆)alkoxy, such as OMe.

According to an embodiment, R and R' are identical.

As compounds of formula (I-6), the followings may be mentioned:

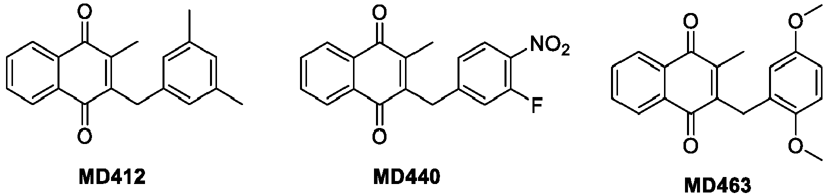

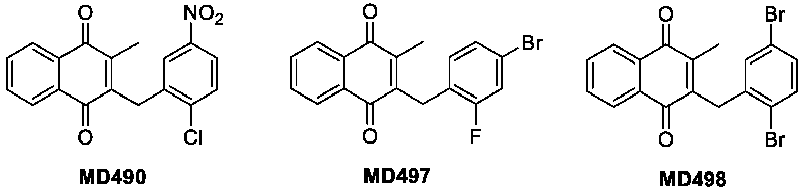

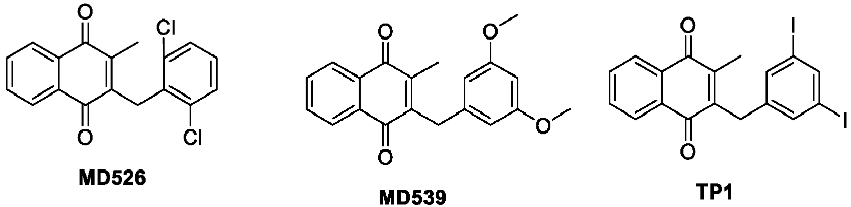

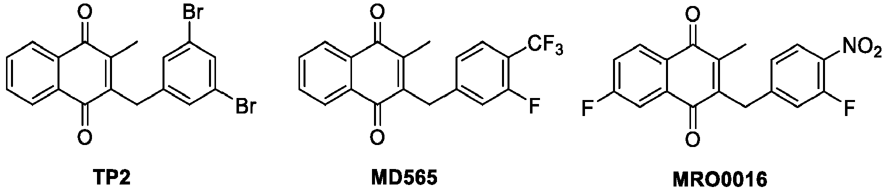

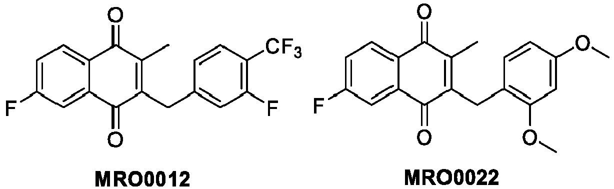

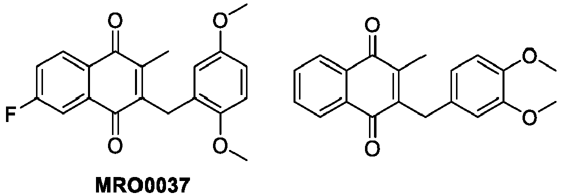

According to an embodiment, a family of compounds prepared by the method according to the present invention consists of compounds having the following formula (I-7): 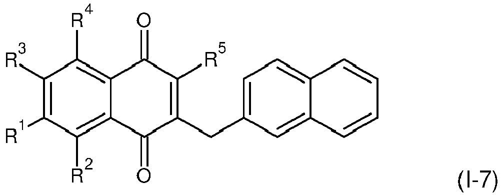 wherein R¹, R², R³, R⁴, and R⁵ are as defined above in formula (I).

A compound of formula (I-7) is for example the following compound:

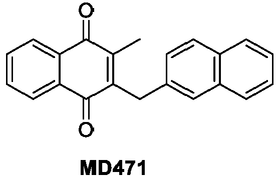

According to an embodiment, a family of compounds prepared by the method according to the present invention consists of compounds having the following formula (I-8): 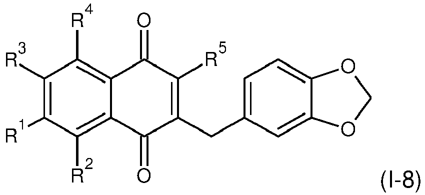 wherein R¹, R², R³, R⁴, and R⁵ are as defined above in formula (I).

As compounds of formula (I-8), the followings may be mentioned:

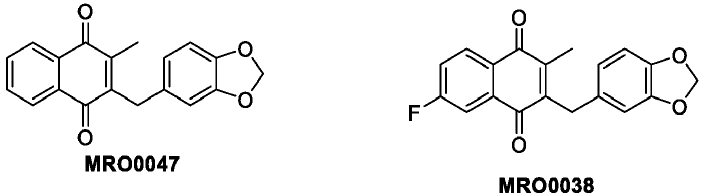

According to an embodiment, in formula (I-1) as defined above, n is 3.

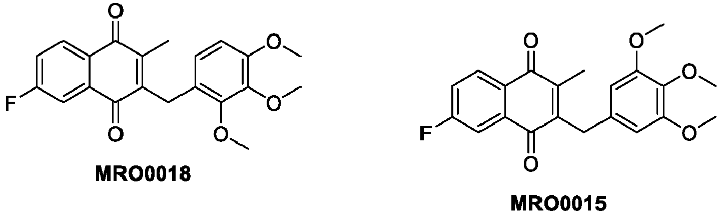

According to an embodiment, in formula (I-1) as defined above, n is 5.

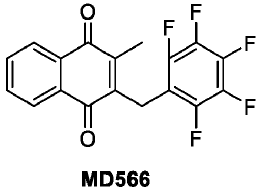

According to an embodiment, a family of compounds prepared by the method according to the present invention consists of compounds having the formula (I) as defined above, wherein R⁵ is selected from the group consisting of:
□ (C₁-C₆)alkyl, such as methyl or ethyl, preferably methyl,
□ halogen, such as Br or Cl,
□ (C₆-C₁₀)aryl, such as phenyl,
□ (C₆-C₁₀)aryloxy, such as phenyloxy,
□ (C₆-C₁₀)aryl(C₁-C₆)alkyl, such as benzyl, and
□ -O-C(=O)-(C₁-C₆)alkyl, such as OAc.

According to an embodiment, a family of compounds prepared by the method according to the present invention consists of compounds having one of the formulae (I'), (I-1), (I-1-1), (I-2), (I-3), (I-4), (I-5), (I-6), (I-7) and (I-8) as defined above, wherein R⁵ is selected from the group consisting of:
□ (C₁-C₆)alkyl, such as methyl or ethyl, preferably methyl,
□ halogen, such as Br or Cl,
□ (C₆-C₁₀)aryl, such as phenyl,
□ (C₆-C₁₀)aryloxy, such as phenyloxy,
□ (C₆-C₁₀)aryl(C₁-C₆)alkyl, such as benzyl, and
□ -O-C(=O)-(C₁-C₆)alkyl, such as OAc.

According to an embodiment, in formula (I), (I'), (I-1), (I-1-1), (I-2), (I-3), (I-4), (I-5), (I-6), (I-7) or (I-8) as defined above, R⁵ is selected from the group consisting of: methyl, ethyl, Br, Cl, phenyl, OAc, phenyloxy, and benzyl.

According to an embodiment, a family of compounds prepared by the method according to the present invention consists of compounds having the formula (I) as defined above, wherein R⁵ is methyl.

According to an embodiment, a family of compounds prepared by the method according to the present invention consists of compounds having the formula (I') as defined above, wherein R⁵ is methyl.

According to an embodiment, a family of compounds prepared by the method according to the present invention consists of compounds having the formula (I-1) or (I-1-1) as defined above, wherein R⁵ is methyl.

According to an embodiment, a family of compounds prepared by the method according to the present invention consists of compounds having the formula (I-2) as defined above, wherein R⁵ is methyl.

According to an embodiment, a family of compounds prepared by the method according to the present invention consists of compounds having the formula (I-3) as defined above, wherein R⁵ is methyl.

According to an embodiment, a family of compounds prepared by the method according to the present invention consists of compounds having the formula (I-4) as defined above, wherein R⁵ is methyl.

According to an embodiment, a family of compounds prepared by the method according to the present invention consists of compounds having the formula (I-5) as defined above, wherein R⁵ is methyl.

According to an embodiment, a family of compounds prepared by the method according to the present invention consists of compounds having the formula (I-6) as defined above, wherein R⁵ is methyl.

According to an embodiment, a family of compounds prepared by the process according to the present invention consists of compounds having the formula (I-7) as defined above, wherein R⁵ is methyl.

According to an embodiment, a family of compounds prepared by the method according to the present invention consists of compounds having the formula (I-8) as defined above, wherein R⁵ is methyl.

According to an embodiment, the present invention relates to a method for the preparation of compounds having the formula (I), wherein:
- R¹ is selected from the group consisting of: H, halogen, -O(P=O)OAlk₂, Alk being as defined above, such as -O(P=O)OEt₂, halo(Ci-Ce)alkyl such as -CF₃, halo(C₁-C₆)alkoxy such as OCF₃, and (C₁-C₆)alkoxy such as OMe;
- R² is selected from the group consisting of: H, OH, and (C₁-C₆)alkoxy; and
- R³ and R⁴ are H.

According to an embodiment, a family of compounds prepared by the method according to the present invention consists of compounds having the following formula (I-9): 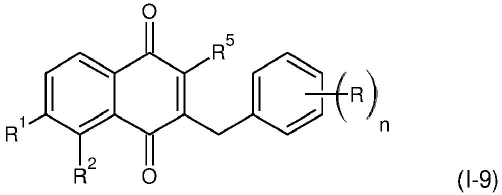 wherein R¹, R², R⁵, R, and n are as defined above.

Preferably, in formula (I-9), R¹ is selected from the group consisting of: H, halogen, -O(P=O)OAlk₂, Alk being as defined above, such as -O(P=O)OEt₂, halo(C₁-C₆)alkyl such as -CF₃, halo(C₁-C₆)alkoxy such as OCF₃, and (C₁-C₆)alkoxy such as OMe.

Preferably, in formula (I-9), R² is selected from the group consisting of: H, OH, and (C₁-C₆)alkoxy.

Preferably, in formula (I-9), R¹ is selected from the group consisting of: H, halogen, -O(P=O)OAlk₂, Alk being as defined above, such as -O(P=O)OEt₂, halo(C₁-C₆)alkyl such as -CF₃, halo(C₁-C₆)alkoxy such as OCF₃, and (C₁-C₆)alkoxy such as OMe, and R² is selected from the group consisting of: H, OH, and (C₁-C₆)alkoxy.

According to an embodiment, the present invention relates to a method for the preparation of compounds having the formula (I'), (I-1), (1-1-1), (I-2), (I-3), (I-4), (I-5), (I-6), (I-7) or (I-8) as defined above, wherein:
- R¹ is selected from the group consisting of: H, halogen, -O(P=O)OAlk₂, Alk being as defined above, such as -O(P=O)OEt₂, halo(C₁-C₆)alkyl such as -CF₃, halo(C₁-C₆)alkoxy such as OCF₃, and (C₁-C₆)alkoxy such as OMe;
- R² is selected from the group consisting of: H, OH, and (C₁-C₆)alkoxy; and
- R³ and R⁴ are H.

According to an embodiment, in formula (I) or (I-9):
- R¹ is H or F, and
- R² is selected from the group consisting of: H, OH, and (C₁-C₆)alkoxy.

According to an embodiment, the present invention relates to a method for the preparation of compounds having the formula (I'), (I-1), (I-1-1), (I-2), (I-3), (I-4), (I-5), (I-6), (I-7) or (I-8) as defined above, wherein:
- R¹ is H or F, and
- R² is selected from the group consisting of: H, OH, and (C₁-C₆)alkoxy.

According to an embodiment, in formula (I) or (I-9):
- R¹ is H or F,
- R² is selected from the group consisting of: H, OH, and (C₁-C₆)alkoxy, and
wherein R² is H when R¹ is F.

Preferably, in formula (I-9), R¹ is F and R² is H.

Preferably, in formula (I-9), R¹ is H and R² is selected from the group consisting of: H, OH, and (C₁-C₆)alkoxy.

According to an embodiment, the present invention relates to a method for the preparation of compounds having the formula (I'), (I-1), (I-1-1), (I-2), (I-3), (I-4), (I-5), (I-6), (I-7) or (I-8) as defined above, wherein:
- R¹ is H or F, and
- R² is selected from the group consisting of: H, OH, and (C₁-C₆)alkoxy, and
wherein R² is H when R¹ is F.

According to an embodiment, a family of compounds prepared by the method according to the present invention consists of compounds having the formula (I) as defined above, wherein R¹, R², R³ and R⁴ are H.

According to an embodiment, a family of compounds prepared by the process according to the present invention consists of compounds having the formula (I) as defined above, wherein R¹ is F, and R², R³ and R⁴ are H.

According to an embodiment, a family of compounds prepared by the method according to the present invention consists of compounds having one of the formulae (I'), (I-1), (I-1-1), (I-2), (I-3), (I-4), (I-5), (I-6), (I-7) and (I-8) as defined above, wherein R¹, R², R³ and R⁴ are H.

According to an embodiment, a family of compounds prepared by the method according to the present invention consists of compounds having one of the formulae (I'), (I-1), (I-1-1), (I-2), (I-3), (I-4), (I-5), (I-6), (I-7) and (I-8) as defined above, wherein R¹ is F, and R², R³ and R⁴ are H.

According to an embodiment, a family of compounds prepared by the method according to the present invention consists of compounds having the following formula (I-10): 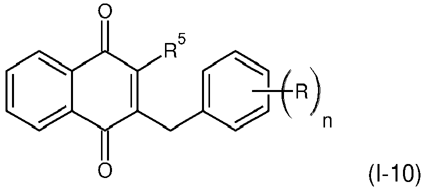 wherein R⁵, R, and n are as defined above in formula (I).

According to an embodiment, a family of compounds prepared by the method according to the present invention consists of compounds having the following formula (I-11): 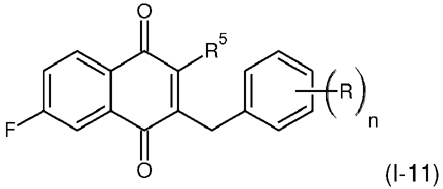 wherein R⁵, R, and n are as defined above in formula (I).

According to an embodiment, a family of compounds prepared by the method according to the present invention consists of compounds having the following formula (I-12): 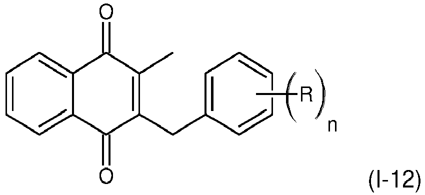 wherein R and n are as defined above in formula (I).

According to an embodiment, a family of compounds prepared by the method according to the present invention consists of compounds having the following formula (I-12-1): 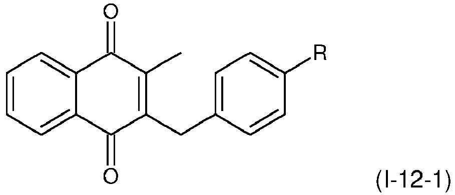 wherein R is as defined above in formula (I).

According to an embodiment, a family of compounds prepared by the method according to the present invention consists of compounds having the following formula (I-13): 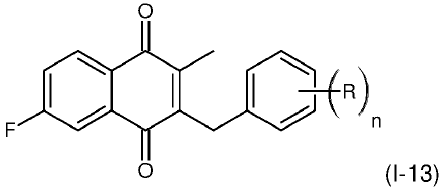 wherein R and n are as defined above in formula (I).

According to an embodiment, a family of compounds prepared by the method according to the present invention consists of compounds having the following formula (I-13-1): 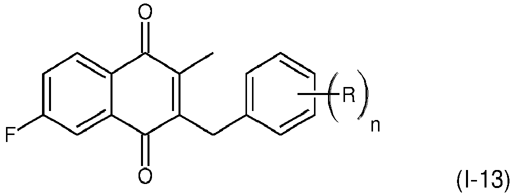 wherein R and n are as defined above in formula (I).

According to an embodiment, a family of compounds prepared by the method according to the present invention consists of compounds having the following formula (I-13-1): 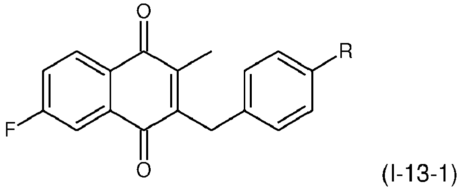 wherein R is as defined above in formula (I).

As explained above, the process of the invention are based on the use of a compound of formula (IV) being CH₂=CH-CH₂-X, preferably CH₂=CH-CH₂-Br, or being a compound of formula (II-1).

Preferably, the method of the invention involves the use of a compound of formula (II-2), that is to say benzyl bromides. These compounds are common building blocks in organic chemistry, widely available and known for their ability to generate after reduction of C-Br bond useful benzyl radical intermediates.

The method of the invention is based on the fruitful combination of an iron catalyst, in particular an iron(III) catalyst, light and quinone, preferably under air atmosphere.

The method of the invention is preferably carried out under blue light irradiation provided by blue LED source (in particular under the form of a 5 meters-long ribbon of a total 24 watts-LEDs). Preferably, the method of the invention is carried out under blue light irradiation at a wavelength comprised between 420 nm and 470 nm.

According to a preferred embodiment, the reacting step is carried out under blue light irradiation at 420 nm.

According to an embodiment of the method, the reacting step as mentioned above is carried out at a temperature comprised from 80°C to 150°C, preferably at 90°C.

According to an embodiment of the method, the reacting step as mentioned above is carried out for a time comprised from 12 hours to 72 hours, preferably for 24 hours.

According to an embodiment of the method, the iron catalyst is a Fe(II) or Fe(III) catalyst. Preferably, the iron catalyst is selected from the group consisting of: FeCl₂, FeCl₃, Fe(acac)₂, Fe(acac)₃, Fe(ClO₄)₃, Fe(NO₃)₃, and ((NH₄)₂Fe(SO₄)₂-6H₂O).

According to an embodiment, the iron catalyst is a Fe(III) catalyst, and is preferably Fe(acac)₃.

According to the invention, the term "heteroatomic base" refers to a base comprising one or several heteroatoms.

According to an embodiment, the heteroatomic base is selected from the group consisting of: pyridine, picoline, 2,6-lutidine, collidine, and DIPEA, and is preferably 2,6-lutidine.

According to the invention, the term "HAT agent" refers to an agent for hydrogen atom transfer.

According to an embodiment, the HAT agent is selected from the group consisting of: Hantzsch's esters, analogues thereof, and γ-terpinene, and is preferably γ-terpinene.

According to an embodiment, the reacting step is carried out in a solvent.

Preferably, the reacting step is carried out in a solvent selected from the group consisting of: acetonitrile, toluene, hexafluoroisopropanol, dichloroethane, dimethylformamide, ethyl acetate, and isopropanol, said solvent being preferably acetonitrile.

According to an embodiment of the method of the invention, the amount of catalyst is comprised between 2% and 20% in moles in relation to the number of moles of compound of formula (III) as defined above, and is preferably 10% in moles in relation to the number of moles of compound of formula (III).

According to an embodiment of the method of the invention, the amount of heteroatomic base is comprised from 1 to 5 equivalents, and is preferably equal to 1.2 equivalent, in relation to the amount of compound of formula (III) as defined above.

According to an embodiment of the method of the invention, the amount of HAT agent is comprised from 0.2 to 5 equivalents, and is preferably equal to 1.2 equivalent, in relation to the amount of compound of formula (III) as defined above.

According to an embodiment of the method of the invention, the amount of compound of formula (IV) is comprised from 1 to 5 equivalents, and is preferably equal to 1.5 equivalent, in relation to the amount of compound of formula (III).

The present invention also relates to a compound having the formula (V):

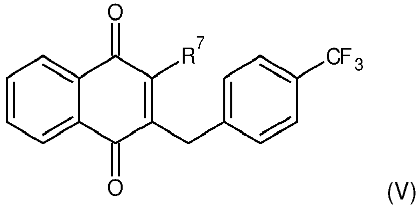

R⁷ being selected from the group consisting of:
□ halogen, such as Cl or Br,
□ (C₆-C₁₀)aryl, such as Ph,
□ (C₆-C₁₀)aryloxy, such as OPh, and
□ (C₆-C₁₀)aryl(C₁-C₆)alkyl, such as benzyl,

Preferably, the compound of formula (V) is selected from the following compounds:

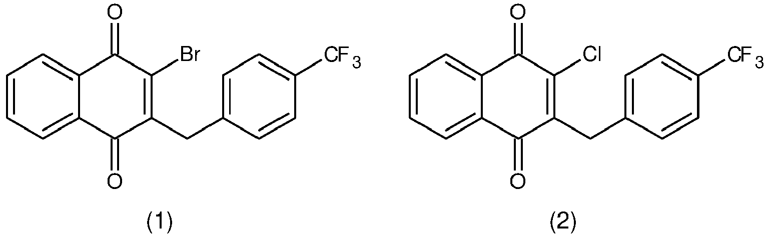

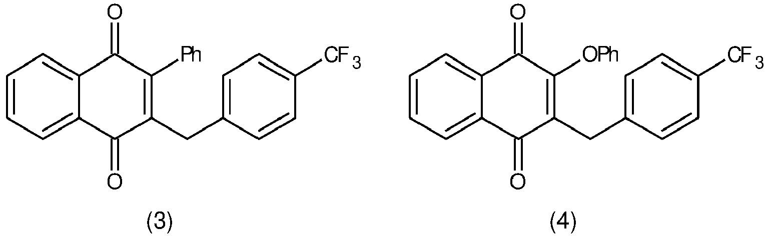

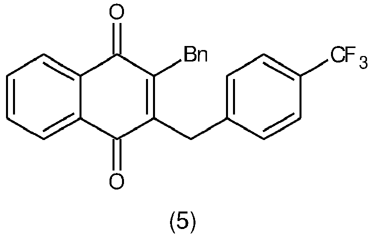

### EXAMPLES

### General information

Solvents and reagents: Commercially available starting materials were purchased from Sigma-Aldrich, ABCR GmbH & Co. KG, Alfa Aesar, and Apollo Scientific and were used without further purification. Solvents were obtained from Sigma-Aldrich and LPCR. All reactions were performed in standard glassware. Thin Layer Chromatography (TLC) were used to monitor reactions (vide infra). Crude mixtures were purified by flash column chromatography. The latter were performed using silica gel 60 (230-400 mesh, 0.040-0.063 mm) purchased from E. Merck. Automatic flash chromatographies were carried out in a Biotage Puriflash apparatus with UV-Vis detection at 254 nm (unless otherwise specified). Monitoring and primary characterization of products were achieved by Thin Layer Chromatography on aluminum sheets coated with silica gel 60 F254 purchased from E. Merck. Eluted TLC's were revealed under UV (325 nm and 254 nm) and with chemicals. Analytical TLC was carried out on pre-coated Sil G-25 UV₂₅₄ plates from Macherey Nagel. Flash chromatography was performed using silica gel G60 (230-400 mesh) from E. Merck. Nuclear.

Magnetic Resonance (NMR): The Nuclear Magnetic Resonance (NMR) spectra were recorded by a Bruker Avance 400 apparatus (¹H NMR 400 MHz, ¹³C NMR 100 MHz, ¹⁹F NMR 377 MHz) or Bruker Avance III HD - 500 MHz apparatus (¹H NMR 500 MH_{z}, ¹³C NMR 125 MHz, ¹⁹F NMR 471 MHz) at the ECPM. All chemical shifts (δ) are quoted in parts per million (ppm). The chemical shifts are referred to the used partial deuterated NMR solvent (for CDCl₃: ¹H NMR, 7.26 ppm and ¹³C NMR, 77.00 ppm; for MeCN: ¹H NMR, 1.93 ppm and ¹³C NMR 1.3 and 118.2 ppm). The coupling constants (J) and the non equivalence (Δv) are given in Hertz (Hz). Resonance patterns are reported with the following notations: br (broad), s (singlet), d (doublet), t (triplet), q (quartet), m (multiplet), dd (doublet of doublets), AB (AB system), (ABX) (AB system of an ABX) and A₂B₂ (A₂B₂ aromatic system). In addition, the following acronyms will be used: C=O carbonyl group; C_{q}: quaternary carbon; CH₂: secondary carbon; CH₃: methyl group.

Microanalyses: Microanalyses were obtained at *"Service de Microanalyses"* at the Institute de Chimie de Strasbourg.

Mass spectrometry: Mass spectra (ESI-MS) were obtained on a microTOF LC spectrometer (Bruker Daltonics, Bremen). High Resolution Mass (HRMS) spectra were measured and fitted with calculated data.

Melting point: Melting points were determined on a Büchi melting point apparatus and were not corrected.

### General procedure for the photocatalytic benzylation of quinones:

In a 10 mL tube, quinone (0.5 mmol), benzyl bromide (0.75 mmol) and Fe(acac)₃ (10%) were dissolved in Acetonitrile (5 mL). The mixture was put under agitation and 2,6-lutidine (0.6 mmol) and γ-terpinene (0.6 mmol) were added successively. The tube was sealed, put under blue light irradiation and heated up at 90°C during 24h. After completion, the mixture was allowed to cool down at room temperature and was partitioned between ethyl acetate (10 mL) and aqueous 1M HCl (10 mL). The aqueous layer was extracted once with 10 mL ethyl acetate and the reunited organic layers were washed with brine and dried with MgSO₄. The solvent was removed and the crude was purified by silica gel chromatography to obtained pure benzylated quinone.

### Example 1: Preparation of 2-methyl-3-(4-(trifluoromethyl)benzoyl) naphthalene-1,4-dione (MD272):

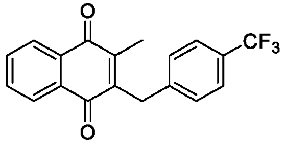

According to the general procedure, menadione and 4-trifluoromethylbenzyl bromide were used. After chromatography on silica gel (Cyclohexane:Toluene = 1:1, UV), 139 mg (0.42 mmol, 84% yield) of **MD272** were isolated as a yellow solid. ¹H **NMR (500 MHz, Chloroform-d)** δ 8.13 - 8.02 (m, 2H), 7.74 - 7.65 (m, 2H), 7.52 (d, *J* = 8.1 Hz, 2H), 7.35 (d, *J* = 8.0 Hz, 2H), 4.08 (s, 2H), 2.25 (s, 3H). **¹⁹F NMR (471 MHz, Chloroform-*d*) δ** -62.45. **¹³C NMR (126 MHz, Chloroform-d) δ** 185.22, 184.60, 144.99, 144.51, 142.34, 133.78, 133.76, 132.19, 132.01, 129.01, 128.94 (q, *J*=32.3 Hz), 126.64, 126.51, 125.70 (q, *J*=3.8Hz), 124.26 (q, *J* = 271.8 Hz), 32.47, 13.47.

### Example 2: Preparation of 4-(3-methyl-1,4-dioxo-1,4-dihydronaphthalene-2-carbonyl)benzonitrile (MD402)

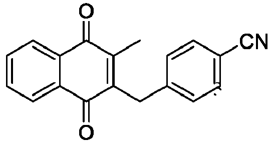

According to the general procedure, menadione and 4-cyannobenzyl bromide were used. After chromatography on silica gel (Toluene, UV), 108 mg (0.38 mmol, 75% yield) of **MD402** were isolated as a yellow solid. **¹H NMR (400 MHz, Chloroform-*d*) δ** 8.15 - 8.01 (m, 2H), 7.82 - 7.66 (m, 2H), 7.63 - 7.50 (m, 2H), 7.41 - 7.30 (m, 2H), 4.08 (s, 2H), 2.24 (s, 3H).**¹³C NMR (101 MHz, Chloroform-*d*) δ** 185.11, 184.55, 145.22, 144.04, 143.85, 133.91, 133.86, 132.60, 132.18, 131.95, 129.49, 126.68, 126.60, 118.88, 110.62, 32.82, 13.55.

### Example 3: Preparation of 2-benzyl-3-methylnaphthalene-1,4-dione (MD404)

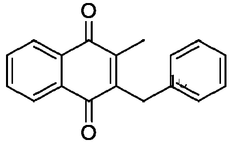

According to the general procedure, menadione and benzyl bromide were used. After chromatography on silica gel (Cyclohexane:Toluene = 4:6, UV), 90 mg (0.34 mmol, 69% yield) of **MD404** were isolated as a yellow solid. **¹H NMR (400 MHz, Chloroform-*d*) δ** 8.14-8.03 (m, 2H), 7.70 (dd, *J* = 5.7, 3.3 Hz, 2H), 7.44-6.92 (m, 5H), 4.04 (s, 2H), 2.25 (s, 3H). **¹³C NMR (101 MHz, Chloroform-*d*) δ** 185.53, 184.79, 145.48, 144.57, 138.19, 133.63, 133.60, 132.27, 132.18, 128.79, 128.73, 126.62, 126.56, 126.41, 32.55, 13.41.

### Example 4: Preparation of 2-(3,5-dimethylbenzyl)-3-methylnaphthalene-1,4-dione (MD412)

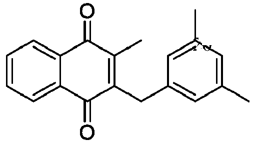

According to the general procedure, menadione and 3,5-dimethylbenzyl bromide were used. After chromatography on silica gel (Cyclohexane:Toluene = 4:6, UV), 101 mg (0.35 mmol, 70% yield) of **MD412** were isolated as a yellow solid. **¹H NMR (400 MHz, Chloroform-*d*) δ** 8.15 - 8.00 (m, 2H), 7.70 (dd, *J* = 5.8, 3.3 Hz, 2H), 7.02 - 6.63 (m, 3H), 3.96 (s, 2H), 2.26 (s, 6H), 2.25 (s, 3H). **¹³C NMR (101 MHz, Chloroform-*d*) δ** 185.64, 184.85, 145.65, 144.54, 138.31, 138.00, 133.60, 133.56, 132.32, 132.25, 128.26, 126.67, 126.47, 126.39, 32.39, 21.44, 13.45.

### Example 5: Preparation of 2-methyl-3-(4-nitrobenzyl)naphthalene-1,4-dione (MD417)

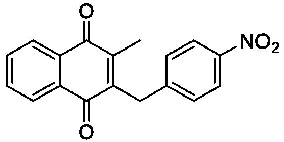

According to the general procedure, menadione and 4-nitrobenzyl bromide were used. After chromatography on silica gel (Cyclohexane:Ethyl acetate = 9:1, UV), 118 mg (0.38 mmol, 77% yield) of **MD417** were isolated as a yellow solid. **¹H NMR (400 MHz, Chloroform-*d*) δ** 8.09 - 7.96 (m, 4H), 7.70 - 7.60 (m, 2H), 7.38 - 7.28 (m, 2H), 4.06 (s, 2H), 2.19 (s, 3H). **¹³C NMR (101 MHz, Chloroform-*d*) δ** 185.08, 184.53, 146.83, 145.97, 145.31, 143.96, 133.95, 133.90, 132.18, 131.94, 129.53, 126.71, 126.63, 124.05, 32.64, 13.59.

### Example 6: Preparation of 2-(4-fluorobenzyl)-3-methylnaphthalene-1,4-dione (MD420)

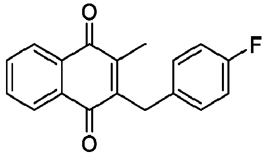

According to the general procedure, menadione and 4-fluorobenzyl bromide were used. After chromatography on silica gel (Cyclohexane:Toluene = 3:7, UV), 93 mg (0.33 mmol, 66% yield) of **MD420** were isolated as a yellow solid. **¹H NMR (400 MHz, Chloroform-d) δ** 8.15 - 8.04 (m, 2H), 7.76 - 7.66 (m, 2H), 7.22 - 7.16 (m, 2H), 7.00 - 6.90 (m, 2H), 4.00 (s, 2H), 2.25 (s, 3H). **¹⁹F NMR (377 MHz, Chloroform-*d*) δ** -116.52 - -116.64 (m). **¹³C NMR (126 MHz, Chloroform-*d*) δ** 185.40, 184.72, 161.62 (d, *J* = 244.7 Hz), 145.23, 144.49, 133.81 (d, *J* = 3.3 Hz), 133.66, 132.20, 132.09, 130.17 (d, *J* = 7.8 Hz), 126.59, 126.43, 115.56 (d, *J* = 21.3 Hz), 31.77, 13.37.

### Example 7: Preparation of methyl 4-((3-methyl-1,4-dioxo-1,4-dihydronaphthalen-2-yl)methyl)benzoate (MD422)

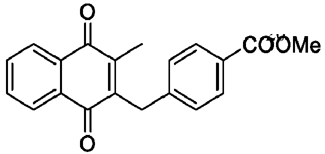

According to the general procedure, menadione and methyl 4-(bromomethyl)benzoate were used. After chromatography on silica gel (Toluene, UV), 80 mg (0.25 mmol, 50% yield) of **MD422** were isolated as a yellow solid. **¹H NMR (400 MHz, Chloroform-*d*) δ** 8.12 - 8.06 (m, 2H), 7.95 - 7.91 (m, 2H), 7.74 - 7.68 (m, 2H), 7.29 (d, J = 8.5 Hz, 2H), 4.08 (s, 2H), 3.88 (s, 3H), 2.23 (s, 3H). **¹³C NMR (101 MHz, Chloroform-d) δ** 185.30, 184.63, 166.98, 145.00, 144.68, 143.61, 133.75, 132.23, 132.06, 130.11, 128.70, 128.57, 126.66, 126.51, 52.18, 32.65, 13.48.

### Example 8: Preparation of 2-(3-fluoro-4-nitrobenzyl)-3-methylnaphthalene-1,4-dione (MD440)

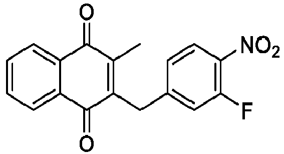

According to the general procedure, menadione and 4-nitro-3-fluorobenzyl bromide were used. After chromatography on silica gel (Cyclohexane:Toluene = 4:6, UV), 91 mg (0.28 mmol, 56% yield) of **MD440** were isolated as a yellow solid. **¹H NMR (400 MHz, Chloroform-d) δ** 8.16 - 8.05 (m, 2H), 7.99 (dd, *J* = 8.7, 7.7 Hz, 1H), 7.82 - 7.65 (m, 2H), 7.22 - 7.11 (m, 2H), 4.10 (s, 2H), 2.26 (s, 3H). **¹⁹F NMR (377 MHz, Chloroform-d) δ** -116.59 (m). **¹³C NMR (101 MHz, Chloroform-*d*) δ** 184.92, 184.41, 155.82 (d, *J* = 265.6 Hz), 147.72 (d, *J* = 8.0 Hz), 145.58, 143.24, 135.94, 134.07, 133.99, 132.16, 131.85, 126.73 (d, *J* = 4.0 Hz), 126.56 (d, *J* = 2.5 Hz), 124.80, 124.76, 118.48 (d, *J* = 21.3 Hz), 32.58 (d, *J* = 1.4 Hz), 13.63.

### Example 9: Preparation of 2-(4-iodobenzyl)-3-methylnaphthalene-1,4-dione (MD444)

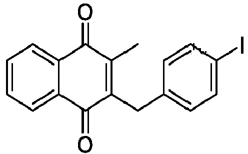

According to the general procedure, menadione and 4-iodobenzyl bromide were used. After chromatography on silica gel (Cyclohexane:Toluene = 4:6, UV), 158 mg (0.407 mmol, 81 % yield) of **MD444** were isolated as a yellow solid. **¹H NMR (400 MHz, Chloroform-*d*) δ** 8.18 - 7.99 (m, 2H), 7.79 - 7.64 (m, 2H), 7.60 - 7.47 (m, 2H), 7.03 - 6.86 (m, 2H), 3.97 (s, 2H), 2.24 (s, 3H). **¹³**C **NMR (101 MHz, Chloroform-*d*) δ** 185.35, 184.69, 144.86, 144.74, 137.91, 137.84, 133.73, 132.23, 132.08, 130.78, 126.65, 126.50, 91.81, 32.17, 13.46.

### Example 10: Preparation of 2-methyl-3-(2-methylbenzyl)naphthalene-1,4-dione (MD454)

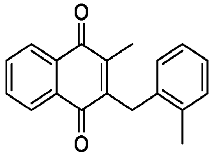

According to the general procedure, menadione and 2-methylbenzyl bromide were used. After chromatography on silica gel (Cyclohexane:Toluene = 4:6, UV), 92 mg (0.33 mmol, 67% yield) of **MD454** were isolated as a yellow solid. **¹H NMR (400 MHz, Chloroform-*d*) δ** 8.15 - 8.11 (m, 1H), 8.11 -8.06 (m, 1H), 7.77 - 7.67 (m, 2H), 7.20 (dd, *J* = 7.3, 1.6 Hz, 1H), 7.11 (td, *J* = 7.4, 1.5 Hz, 1H), 7.04 (td, *J* = 7.6, 1.6 Hz, 1H), 6.84 (dd, *J* = 7.6, 1.3 Hz, 1H), 3.97 (s, 2H), 2.45 (s, 3H), 2.16 (s, 3H). **¹³C NMR (101 MHz, Chloroform-*d*) δ** 185.32, 184.58, 145.65, 145.49, 136.39, 136.14, 133.65, 133.61, 132.38, 132.26, 130.47, 126.73, 126.68, 126.51, 126.47, 126.27, 29.70, 20.12, 13.36.

### Example 11: Preparation of 2-methyl-3-(4-(trifluoromethoxy)benzyl) naphthalene-1,4-dione (MD456)

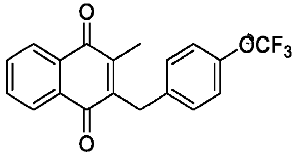

According to the general procedure, menadione and 4-trifluoromethoxybenzyl bromide were used. After chromatography on silica gel (Cyclohexane:Toluene = 4:6, UV), 138 mg (0.4 mmol, 80% yield) of **MD456** were isolated as a yellow solid. **¹H NMR (400 MHz, Chloroform-*d*) δ** 8.12 - 8.05 (m, 2H), 7.71 (dd, *J* = 5.8, 3.3 Hz, 2H), 7.30 - 7.22 (m, 2H), 7.11 (dt, *J* = 7.7, 1.0 Hz, 2H), 4.03 (s, 2H), 2.26 (s, 3H). **¹⁹F NMR (377 MHz, Chloroform-d) δ** -57.93. **¹³C NMR (101 MHz, Chloroform-*d*) δ** 185.36, 184.71, 147.93 (q, *J* = 2.0 Hz), 144.90, 144.74, 136.92, 133.75, 132.24, 132.09, 130.04, 129.18, 128.37, 126.65, 126.51, 120.59 (d, *J* = 256.9 Hz), 31.96, 13.46.

### Example 12: Preparation of 2-(2,5-dimethoxybenzyl)-3-methylnaphthalene-1,4-dione (MD463)

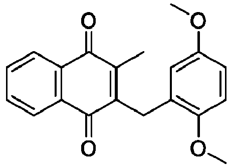

According to the general procedure, menadione and 2,5-dimethoxybenzyl bromide were used. After chromatography on silica gel (Toluene, UV), 128 mg (0.39 mmol, 78% yield) of **MD463** were isolated as a yellow solid. **¹H NMR (400 MHz, Chloroform-*d*) δ** 8.14 - 8.01 (m, 2H), 7.80 - 7.61 (m, 2H), 6.78 (d, *J* = 8.8 Hz, 1 H), 6.69 (dd, *J* = 8.8, 3.1 Hz, 1H), 6.62 (d, *J* = 3.0 Hz, 1H), 4.00 (s, 2H), 3.80 (s, 3H), 3.69 (s, 3H), 2.17 (s, 3H). **¹³C NMR (101 MHz, Chloroform-*d*) δ** 185.54, 184.73, 153.68, 151.67, 145.57, 145.13, 133.54, 133.47, 132.38, 132.37, 127.78, 126.61, 126.38, 116.36, 111.28, 111.05, 56.10, 55.78, 26.85, 13.18.

### Example 13: Preparation of 2-methyl-3-(naphthalen-2-ylmethyl) naphthalene-1,4-dione (MD471)

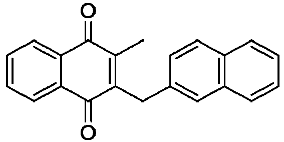

According to the general procedure, menadione and 2-(bromomethyl)naphthalene were used. After chromatography on silica gel (Cyclohexane:Toluene = 4:6, UV), 107 mg (0.34 mmol, 69% yield) of **MD420** were isolated as a yellow solid. **¹H NMR (400 MHz, Chloroform-*d*) δ** 8.20 - 8.04 (m, 2H), 7.85 - 7.67 (m, 5H), 7.64 - 7.60 (m, 1H), 7.51 - 7.34 (m, 3H), 4.21 (s, 2H), 2.29 (s, 3H). **¹³C NMR (101 MHz, Chloroform-*d*) δ** 185.55, 184.85, 145.37, 144.81, 135.68, 133.68, 133.65, 132.32, 132.30, 132.22, 128.48, 127.74, 127.68, 127.27, 126.93, 126.69, 126.46, 126.26, 125.70, 32.72, 13.50.

### Example 14: Preparation of 2-(2-chloro-5-nitrobenzyl)-3-methylnaphthalene-1,4-dione (MD490)

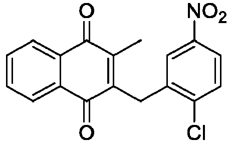

According to the general procedure, menadione and 2-chloro-5-nitrobenzyl bromide were used. After chromatography on silica gel (Cyclohexane:Toluene = 4:6, UV), 119 mg (0.35 mmol, 70% yield) of **MD490** were isolated as a yellow solid. **¹H NMR (400 MHz, Chloroform-*d*) δ** 8.15 - 8.12 (m, 1H), 8.10 - 8.05 (m, 1H), 8.03 (dd, *J* = 8.8, 2.7 Hz, 1H), 7.85 (d, *J* = 2.7 Hz, 1H), 7.79 - 7.68 (m, 2H), 7.57 (d, *J* = 8.7 Hz, 1H), 4.17 (s, 2H), 2.19 (s, 3H). **¹³C NMR (101 MHz, Chloroform-*d*) δ** 184.78, 184.32, 146.87, 146.61, 142.79, 141.06, 137.96, 134.01, 133.95, 132.24, 131.92, 130.58, 126.77, 126.72, 123.97, 122.87, 30.49, 13.61.

### Example 15: Preparation of 2-(4-bromo-2-fluorobenzyl)-3-methylnaphthalene-1,4-dione (MD497)

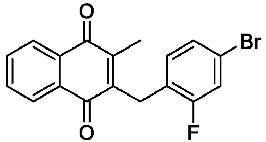

According to the general procedure, menadione and 4-bromo-2-fluorobenzyl bromide were used. After chromatography on silica gel (Cyclohexane:Toluene = 4:6, UV), 129 mg (0.36 mmol, 72% yield) of **MD497** were isolated as a yellow solid. **¹H NMR (500 MHz, Chloroform-*d*) δ** 8.13 - 8.00 (m, 2H), 7.81 - 7.58 (m, 2H), 7.21 (dd, *J* = 9.6, 2.0 Hz, 1H), 7.16 (dd, *J* = 8.3, 2.0 Hz, 1H), 7.06 (t, *J* = 8.1 Hz, 1H), 3.98 (s, 2H), 2.21 (s, 3H). **¹⁹F NMR (471 MHz, Chloroform-*d*) δ** -113.57 (t, *J* = 8.7 Hz). **¹³C NMR (126 MHz, Chloroform-*d*) δ** 185.19, 184.55, 160.70 (d, *J* = 250.3 Hz), 145.45, 143.75, 133.75, 132.22, 132.07, 131.74 (d, *J* = 4.9 Hz), 127.65 (d, *J* = 3.6 Hz), 126.64, 126.54, 124.31 (d, *J* = 15.7 Hz), 120.53 (d, *J* = 9.6 Hz), 119.17 (d, *J* = 25.6 Hz), 25.52 (d, *J* = 3.1 Hz), 13.21 (d, *J* = 2.7 Hz).

### Example 16: Preparation of 2-(2,5-dibromobenzyl)-3-methylnaphthalene-1,4-dione (MD498)

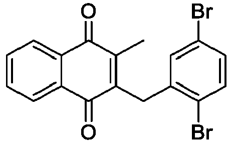

According to the general procedure, menadione and 2,5-dibromobenzyl bromide were used. After chromatography on silica gel (Cyclohexane:Toluene = 4:6, UV), 156 mg (0.37 mmol, 74% yield) of **MD490** were isolated as a yellow solid. **¹H NMR (400 MHz, Chloroform-*d*) δ** 8.22 - 8.02 (m, 2H), 7.83 - 7.67 (m, 2H), 7.45 (d, *J* = 8.5 Hz, 1H), 7.20 (dd, *J* = 8.5, 2.4 Hz, 1H), 7.00 (d, *J* = 2.3 Hz, 1H), 4.09 (s, 2H), 2.14 (s, 3H). **¹³C NMR (101 MHz, Chloroform-d) δ** 185.04, 184.37, 146.38, 143.84, 139.75, 134.36, 133.87, 132.31, 132.06, 131.56, 131.26, 126.82, 126.64, 123.48, 121.75, 32.81, 13.57.

### Example 17: Preparation of 2-methyl-3-(4-methylbenzyl)naphthalene-1,4-dione (MD510)

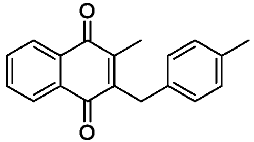

According to the general procedure, menadione and 4-methylbenzyl bromide were used. After chromatography on silica gel (Cyclohexane:Toluene = 4:6, UV), 71 mg (0.26 mmol, 51% yield) of **MD510** were isolated as a yellow solid. **¹H NMR (500 MHz, Chloroform-*d*) δ** 8.08 (m, 2H), 7.69 (m, 2H), 7.13 (d, *J* = 8.2 Hz, 2H), 7.07 (d, *J* = 8.1 Hz, 2H), 4.00 (s, 2H), 2.29 (s, 3H), 2.25 (s, 3H). **¹³C NMR (126 MHz, Chloroform-*d*) δ** 185.59, 184.83, 145.68, 144.36, 136.13, 135.09, 133.59, 133.55, 132.26, 132.21, 129.47, 128.62, 126.60, 126.38, 32.14, 21.12, 13.39.

### Example 18: Preparation of 2-(4-chlorobenzyl)-3-methylnaphthalene-1,4-dione (MD522)

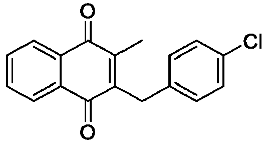

According to the general procedure, menadione and 4-chlorobenzyl bromide were used. After chromatography on silica gel (Cyclohexane:Toluene = 4:6, UV), 109 mg (0.37 mmol, 73% yield) of **MD522** were isolated as a yellow solid. **¹H NMR (400 MHz, Chloroform-*d*) δ** 8.28 - 7.95 (m, 2H), 7.70 (m, 2H), 7.25 - 7.20 (m, 2H), 7.19 - 7.13 (m, 2H), 3.99 (s, 1H), 2.24 (s, 1H). **¹³C NMR (101 MHz, Chloroform-*d*) δ** 185.36, 184.69, 144.97, 144.68, 136.66, 133.72, 132.41, 132.22, 132.08, 130.07, 128.89, 126.63, 126.48, 31.98, 13.43.

### Example 19: Preparation of 2-(4-bromobenzyl)-3-methylnaphthalene-1,4-dione (MD523)

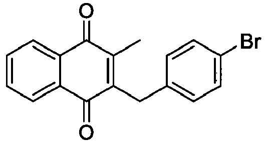

According to the general procedure, menadione and 4-bromobenzyl bromide were used. After chromatography on silica gel (Cyclohexane:Toluene = 4:6, UV), 137 mg (0.402 mmol, 80% yield) of **MD523** were isolated as a yellow solid. **¹H NMR (400 MHz, Chloroform-*d*) δ** 8.19 - 8.01 (m, 2H), 7.70 (m, 2H), 7.44 - 7.35 (m, 2H), 7.16 - 7.04 (m, 2H), 3.97 (s, 2H), 2.24 (s, 3H). **¹³C NMR (101 MHz, Chloroform-*d*) δ** 185.34, 184.68, 144.88, 144.71, 137.20, 133.72, 132.21, 132.07, 131.85, 130.45, 126.63, 126.48, 120.44, 32.05, 13.44.

### Example 20: Preparation of 2-methyl-3-(3-(trifluoromethyl) benzyl)naphthalene-1,4-dione (MD525)

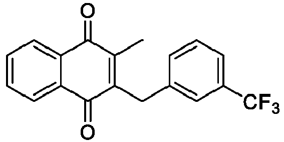

According to the general procedure, menadione and 3-trifluoromethylbenzyl bromide were used. After chromatography on silica gel (Cyclohexane:Toluene = 4:6, UV), 126 mg (0.38 mmol, 76% yield) of **MD525** were isolated as a yellow solid. **¹H NMR (400 MHz, Chloroform-*d*) δ** 8.09 (m, 2H), 7.71 (m, 2H), 7.53 - 7.34 (m, 4H), 4.08 (s, 2H), 2.25 (s, 3H). **¹⁹F NMR (377 MHz, Chloroform-*d*) δ** -62.58. **¹³C NMR (101 MHz, Chloroform-*d*) δ** 185.13, 184.48, 144.86, 144.37, 139.05, 133.64, 132.09, 131.94, 131.92, 131.91, 130.97 (q, *J* = 32.2 Hz), 129.11, 126.55, 126.39, 125.32 (q, *J* = 3.9 Hz), 124.04 (q, *J* = 272.4 Hz), 123.40 (q, *J* = 3.8 Hz), 32.29, 13.35.

### Example 21: Preparation of 2-(2,6-dichlorobenzyl)-3-methylnaphthalene-1,4-dione (MD526)

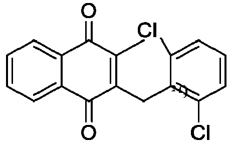

According to the general procedure, menadione and 2,6-dichlorobenzyl bromide were used. After chromatography on silica gel (Cyclohexane:Toluene = 4:6, UV), 126 mg (0.38 mmol, 76% yield) of **MD526** were isolated as a yellow solid. **¹H NMR (400 MHz, Chloroform-*d*) δ** 8.21 - 7.99 (m, 2H), 7.75 - 7.63 (m, 2H), 7.30 (d, *J* = 8.0 Hz, 2H), 7.15 - 7.08 (m, 1 H), 4.37 (s, 2H), 2.04 (s, 3H). **13C NMR (101 MHz, Chloroform-*d*) δ** 185.12, 184.12, 144.58, 144.47, 135.92, 135.46, 133.63, 133.53, 132.24, 132.20, 128.58, 128.30, 126.77, 126.43, 29.48, 12.85.

### Example 22: Preparation of 2-methyl-3-(2-(trifluoromethyl)benzyl)naphthalene-1,4-dione (MD529)

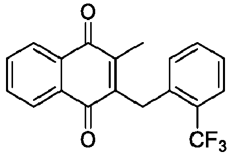

According to the general procedure, menadione and 2-trifluoromethylbenzyl bromide were used. After chromatography on silica gel (Cyclohexane:Toluene = 4:6, UV), 126 mg (0.38 mmol, 76% yield) of **MD529** were isolated as a yellow solid. **¹H NMR (400 MHz, Chloroform-*d*) δ** 8.28 - 8.01 (m, 2H), 7.78 - 7.72 (m, 2H), 7.70 (d, *J* = 7.3 Hz, 1H), 7.36 (t, *J* = 7.1 Hz, 1H), 7.30 (t, *J* = 7.3 Hz, 1H), 6.96 (d, *J* = 7.6 Hz, 1H), 4.25 (s, 2H), 2.11 (s, 3H). **¹⁹F NMR (377 MHz, Chloroform-*d*) δ** -60.90. **¹³C NMR (101 MHz, Chloroform-d) δ** 185.13, 184.50, 146.42, 144.36, 136.75, 133.83, 133.81, 132.36, 132.23, 132.10, 128.69 (q, *J* = 29.9 Hz), 128.29, 126.77, 126.60, 126.56, 126.44 (q, *J* = 5.8 Hz), 124.70 (q, *J* = 273.8 Hz), 28.79 (q, *J* = 2.6 Hz), 13.23.

### Example 23: Preparation of 2-(3,5-dimethoxybenzyl)-3-methylnaphthalene-1,4-dione (MD539)

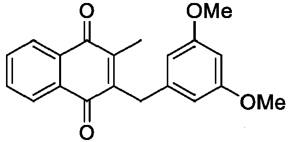

According to the general procedure, menadione and 3,5-dimethoxybenzyl bromide were used. After chromatography on silica gel (Cyclohexane:Ethyl Acetate = 95:5, UV), 110 mg (0.34 mmol, 68% yield) of **MD539** were isolated as a yellow solid. **¹H NMR (400 MHz, Chloroform-*d*) δ** 8.17 - 7.99 (m, 2H), 7.74 - 7.65 (m, 2H), 6.37 (d, *J* = 2.2 Hz, 2H), 6.29 (t, *J* = 2.2 Hz, 1 H), 3.97 (s, 2H), 3.75 (s, 6H), 2.24 (s, 3H). **¹³C NMR (101 MHz, Chloroform-*d*) δ** 185.46, 184.72, 161.04, 145.09, 144.78, 140.42, 133.61, 133.59, 132.27, 132.17, 126.63, 126.40, 106.97, 98.14, 55.39, 32.64, 13.42.

### Example 24: Preparation of 2-(2-bromobenzyl)-3-methylnaphthalene-1,4-dione (MD547)

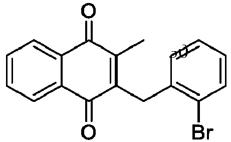

According to the general procedure, menadione and 2-bromobenzyl bromide were used. After chromatography on silica gel (Cyclohexane:Toluene = 4:6, UV), 134 mg (0.39 mmol, 79% yield) of **MD547** were isolated as a yellow solid. **¹H NMR (400 MHz, Chloroform-*d*) δ** 8.19 - 7.99 (m, 2H), 7.79 - 7.64 (m, 2H), 7.59 (dd, *J* = 8.0, 1.4 Hz, 1H), 7.15 (td, *J* = 7.5, 1.4 Hz, 1H), 7.06 (td, *J* = 7.6, 1.8 Hz, 1H), 6.92 (dd, *J* = 7.6, 1.8 Hz, 1H), 4.13 (s, 2H), 2.13 (s, 3H). **¹³C NMR (101 MHz, Chloroform-*d*) δ** 185.20, 184.51, 146.04, 144.73, 137.49, 133.75, 133.72, 133.05, 132.32, 132.15, 128.77, 128.12, 127.73, 126.72, 126.52, 124.84, 32.83, 13.45.

### Example 25: Preparation of 2-(3,5-diiodobenzyl)-3-methylnaphthalene-1,4-dione (TP1)

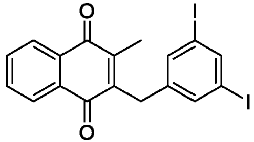

According to the general procedure, menadione and 3,5-diiodobenzyl bromide were used. After chromatography on silica gel (Cyclohexane:Toluene = 1:1, UV), 191 mg (0.37 mmol, 74% yield) of **TP1** were isolated as a yellow solid. **¹H (400MHz, Chloroform-*d*) δ** 8.12 (m, 2H), 7.88 (t, J = 1.8 Hz, 1H), 7.72 (m, 2H), 7.53-7.50 (m, 2H), 3.91 (s, 2H), 2.23 (s, 3H). **¹³C NMR (126 MHz, Chloroform-*d*) δ** 185.17, 184.45, 145.21, 143.90, 143.50, 142.27, 136.92, 133.86, 133.83, 132.22, 131.98, 126.76, 126.59, 95.20, 31.73, 13.61.

### Example 26: Preparation of 2-(3,5-dibromobenzyl)-3-methylnaphthalene-14-dione (TP2)

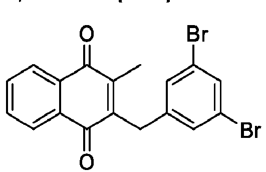

According to the general procedure, menadione and 3,5-dibromobenzyl bromide were used. After chromatography on silica gel (Cyclohexane:Toluene = 1:1, UV), 135 mg (0.32 mmol, 64% yield) of **TP2** were isolated as a yellow solid. **¹H (400MHz, Chloroform-*d*) δ** 8.11 (m, 2H), 7.75-7.71 (m, 2H), 7.51 (t, J = 1.8 Hz, 1H), 7.30 (m, 2H), 3.91 (s, 2H), 2.23 (s, 3H). **¹³C NMR (126 MHz, Chloroform-*d*) δ** 185.17, 184.47, 145.27, 143.88, 142.07, 133.88, 133.86, 132.45, 132.22, 131.98, 130.49, 126.77, 126.61, 123.25, 32.00, 13.59.

### Example 27: Preparation of 2-(3-fluoro-4-(trifluoromethyl)benzyl)-3-methyl naphthalene-1,4-dione (MD565)

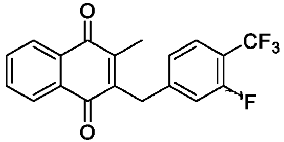

According to the general procedure, menadione and 3-fluro-4-trifluoromethylbenzyl bromide were used. After chromatography on silica gel (Cyclohexane:Toluene = 4:6, UV), 129 mg (0.37 mmol, 74% yield) of **MD565** were isolated as a yellow solid. **¹H NMR (500 MHz, Chloroform-*d*) δ** 8.16 - 8.00 (m, 2H), 7.81 - 7.66 (m, 2H), 7.50 (t, *J* = 7.7 Hz, 1H), 7.12 (dd, *J* = 8.1, 1.2 Hz, 1H), 7.09 - 7.02 (m, 1H), 4.07 (s, 2H), 2.25 (s, 3H). **¹⁹F NMR (471 MHz, Chloroform-*d*) δ** -61.20 (d, *J* = 12.2 Hz), -114.07 (td, *J* = 12.0, 7.3 Hz). **¹³C NMR (126 MHz, Chloroform-*d*) δ** 185.08, 184.50, 159.97 (dq, *J* = 256.4, 2.1 Hz), 145.36, 145.30, 143.80, 133.93, 133.88, 132.19, 131.94, 127.45 (qd, *J* = 4.4, 2.1 Hz), 126.71, 126.61, 124.32 (d, *J* = 3.5 Hz), 122.71 (q, *J* = 272.0 Hz), 117.05 (d, *J* = 21.0 Hz), 116.72 (qd, *J* = 33.2, 12.3 Hz), 32.36, 13.53.

### Example 28: Preparation of 2-methyl-3-((perfluorophenyl) methyl)naphthalene-1,4-dione (MD566)

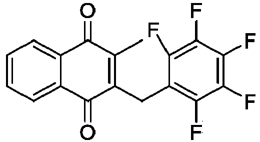

According to the general procedure, menadione and 2,3,4,5,6-pentafluorobenzyl bromide were used. After chromatography on silica gel (Cyclohexane:Toluene = 4:6, UV), 125 mg (0.35 mmol, 71% yield) of **MD566** were isolated as a yellow solid. **¹H NMR (400 MHz, Chloroform-*d*) δ** 8.09 - 8.04 (m, 1H), 8.03 - 7.98 (m, 1H), 7.75 - 7.63 (m, 2H), 4.03 (s, 2H), 2.24 (s, 3H). **¹⁹F NMR (471 MHz, Chloroform-*d*) δ** -141.23 - -141.33 (m), -156.45 (t, *J* = 20.9 Hz), -162.22 - - 162.38 (m). **¹³C NMR (126 MHz, Chloroform-*d*) δ** 184.78, 183.76, 145.54, 145.41 (dm, *J* = 247.0 Hz), 141.96, 140.15 (dm, *J* = 252.7 Hz), 138.81 - 136.39 (dm, *J* = 251.8 Hz), 133.83, 133.80, 132.08, 131.85, 126.57, 126.56, 112.13 (td, *J* = 17.5, 4.1 Hz), 21.01, 13.04.

### Example 29: Preparation of 2-allyl-3-methylnaphthalene-1,4-dione (MD598)

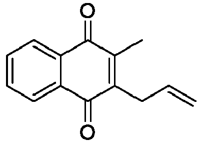

A variant from the general procedure was performed. In a 10 mL tube, quinone (0.5 mmol), allyl bromide (1 mmol) and Fe(acac)₃ (10%) were dissolved in Acetonitrile (5 mL). The mixture was put under agitation and 2,6-lutidine (0.6 mmol) and γ-terpinene (0.6 mmol) were added successively. The tube was sealed, put under blue light irradiation and heated up at 90°C during 24h. After completion, the mixture was allowed to cool down at room temperature and was partitioned between ethyl acetate (10 mL) and aqueous 1M HCl (10 mL). The aqueous layer was extracted once with 10 mL ethyl acetate and the reunited organic layers were washed with brine and dried with MgSO₄. The solvent was removed and the crude was purified by silica gel chromatography (Cyclohexane:Ethyl Acetate = 95:5, UV) to obtained 72 mg (0.34 mmol, 68%) of **MD598** as an orange solid. **¹H NMR (500 MHz, Chloroform-*d*) δ** 8.11 - 8.03 (m, 2H), 7.75 - 7.60 (m, 2H), 5.83 (ddt, *J* = 17.1, 10.1, 6.3 Hz, 1H), 5.15-5.01 (m, 2H), 3.41 (dd, *J* = 6.3, 0.7 Hz, 2H), 2.19 (s, 3H). **¹³C NMR (126 MHz, Chloroform-*d*) δ** 185.37, 184.38, 144.41, 144.40, 133.57, 133.53, 133.32, 132.27, 132.20, 126.50, 126.40, 116.76, 31.05, 12.73.

### Example 30: Preparation of 2-bromo-3-(4-(trifluoromethyl) benzyl)naphthalene-1,4-dione (MD537)(compound 1)

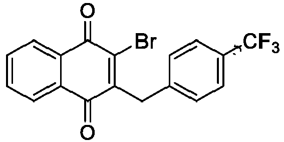

According to the general procedure, 2-bromonaphthalene-1,4-dione and 4-trifluoromethylbenzyl bromide were used. After chromatography on silica gel (Cyclohexane:Toluene = 4:6, UV), 84 mg (0.21 mmol, 43% yield) of **MD537** were isolated as a yellow solid. **¹H NMR (400 MHz, Chloroform-*d*) δ** 8.19 - 8.15 (m, 1H), 8.14 - 8.08 (m, 1H), 7.85 - 7.70 (m, 2H), 7.59 - 7.48 (m, 4H), 4.29 (s, 2H). **¹⁹F NMR (377 MHz, Chloroform-*d*) δ** -62.56. **¹³C NMR (101 MHz, Chloroform-*d*) δ** 181.82, 177.83, 149.18, 140.75, 140.54, 134.54, 134.34, 131.50, 131.23, 129.61, 129.05 (q, *J* = 36.5 Hz), 127.81, 127.47, 125.72 (q, *J* = 3.8 Hz), 124.24 (q, *J* = 272.0 Hz), 36.91.

### Example 31: Preparation of 5-hydroxy-2-methyl-3-(4-(trifluoromethyl) benzyl)naphthalene-1,4-dione (MD536)

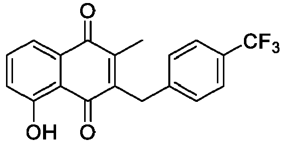

According to the general procedure, Plumbagin and 4-trifluoromethylbenzyl bromide were used. After chromatography on silica gel (Cyclohexane:Toluene = 4:6, UV), 64 mg (0.185 mmol, 37% yield) of **MD536** were isolated as an orange solid. **¹H NMR (400 MHz, Chloroform-*d*) δ** 12.06 (s, 1H), 7.67 - 7.55 (m, 2H), 7.54 (d, *J* = 8.2 Hz, 2H), 7.35 (d, *J* = 7.9 Hz, 2H), 7.24 (dd, *J* = 8.2, 1.4 Hz, 1H), 4.07 (s, 2H), 2.25 (s, 3H). **¹⁹F NMR (471 MHz, CDCl₃) δ** -62.46. **¹³C NMR (126 MHz, CDCl₃) δ** 189.70, 184.40, 161.47, 146.41, 144.30, 141.99, 136.35, 132.15, 129.10 (q, *J* = 32.4 Hz), 128.94, 125.78 (q, *J* = 3.8 Hz), 124.23, 124.22 (q, *J =* 272.1 Hz), 119.26, 114.92, 31.85, 13.59.

### Example 32: Preparation of 5-methoxy-2-methyl-3-(4-(trifluoromethyl)benzyl)naphthalene-1,4-dione (MD546)

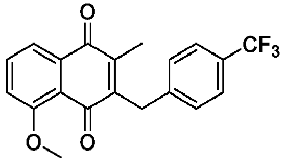

According to the general procedure, 5-methoxy-2-methylnaphthalene-1,4-dione and 4-trifluoromethylbenzyl bromide were used. After chromatography on silica gel (Cyclohexane:Toluene = 4:6, UV), 90 mg (0.25 mmol, 50% yield) of **MD546** were isolated as an orange solid. **¹H NMR (400 MHz, Chloroform-*d*) δ** 7.76 (dd, *J* = 7.6, 1.1 Hz, 1H), 7.64 (dd, *J* = 8.5, 7.7 Hz, 1H), 7.50 (d, *J* = 8.1 Hz, 2H), 7.35 (d, *J* = 7.9 Hz, 2H), 7.27 (d, *J* = 8.5 Hz, 1H), 4.05 (s, 2H), 3.99 (s, 3H), 2.19 (s, 3H). **¹⁹F NMR (377 MHz, Chloroform-*d*) δ** -62.45. **¹³C NMR (101 MHz, Chloroform-*d*) δ** 185.52, 183.83, 159.72, 146.18, 142.65, 142.61, 134.86, 134.44, 129.07, 128.80 (q, *J* = 32.5 Hz), 125.63 (q, *J* = 3.6 Hz), 124.26 (q, *J* = 271.8 Hz), 119.84, 119.30, 117.69, 56.59, 32.67, 13.20.

### Example 33: Preparation of 2-chloro-3-(4-(trifluoromethyl) benzyl)naphthalene-1,4-dione (MD587)(compound 2)

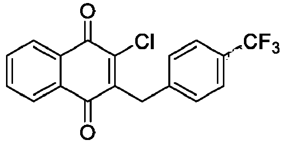

According to the general procedure, 2-chloronaphthalene-1,4-dione and 4-trifluoromethylbenzyl bromide were used. After chromatography on silica gel (Cyclohexane:Toluene = 4:6, UV), 95 mg (0.27 mmol, 54% yield) of **MD587** were isolated as a yellow solid. **¹H NMR (500 MHz, Chloroform-*d*) δ** 8.19 - 8.15 (m, 1H), 8.11 (m, 1H), 7.81 - 7.72 (m, 2H), 7.54 (d, *J* = 8.4 Hz, 2H), 7.49 (d, *J* = 8.3 Hz, 2H), 4.23 (s, 3H). **¹⁹F NMR (471 MHz, Chloroform-*d*)** -62.54. **δ ¹³C NMR (126 MHz, Chloroform-*d*) δ** 182.41, 177.83, 145.66, 144.37, 140.89, 134.59, 134.36, 131.63, 131.43, 129.66, 129.39 (q, *J* = 32.6 Hz), 127.47, 127.37, 125.75 (q, *J* = 3.7 Hz), 124.23 (q, *J* = 272.3 Hz), 33.91.

### Example 34: Preparation of 6-fluoro-2-methyl-3-(4-(trifluoromethyl)benzyl) naphthalene-1,4-dione (MD593)

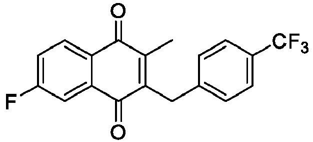

According to the general procedure, 6-fluoro-2-methylnaphthalene-1,4-dione and 4-trifluoromethylbenzyl bromide were used. After chromatography on silica gel (Cyclohexane:Toluene = 1:1, UV), 116 mg (0.33 mmol, 67% yield) of **MD593** were isolated as a yellow solid. **¹H NMR (500 MHz, Chloroform-*d*) δ** 8.13 (dd, *J* = 8.6, 5.2 Hz, 1H), 7.72 (dd, *J* = 8.5, 2.7 Hz, 1H), 7.55-7.48 (m, 2H), 7.40 - 7.30 (m, 3H), 4.08 (s, 2H), 2.26 (s, 3H). **¹⁹F NMR (471 MHz, Chloroform-*d*) δ** -62.49, -102.18 (td, *J* = 8.4, 5.3 Hz). **¹³C NMR (126 MHz, Chloroform-*d*) δ** 183.82, 183.45, 166.05 (d, *J* = 257.1 Hz), 145.18, 144.58 (d, *J* = 1.8 Hz), 141.98, 134.48 (d, *J* = 7.8 Hz), 129.73 (d, *J* = 8.9 Hz), 128.97 (q, *J* = 32.3 Hz), 128.90, 128.69 (d, *J* = 3.3 Hz), 125.66 (q, *J* = 3.8 Hz), 124.13 (q, *J* = 271.9 Hz), 120.90 (d, *J* = 22.5 Hz), 113.26 (d, *J* = 23.5 Hz), 32.41, 13.41.

### Example 35: Preparation of 2-ethyl-3-(4-(trifluoromethyl)benzyl) naphthalene-1,4-dione (MD602)

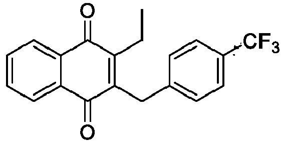

According to the general procedure, 2-ethylnaphthalene-1,4-dione and 4-trifluoromethylbenzyl bromide were used. After chromatography on silica gel (Cyclohexane:Toluene = 1:1, UV), 108 mg (0.31 mmol, 63% yield) of **MD602** were isolated as an orange solid. **¹H NMR (500 MHz, Chloroform-*d*) δ** 8.15 - 7.99 (m, 2H), 7.78 - 7.65 (m, 2H), 7.52 (d, *J* = 8.0 Hz, 2H), 7.34 (d, *J* = 8.0 Hz, 2H), 4.09 (s, 2H), 2.72 (q, *J* = 7.6 Hz, 2H), 1.09 (t, *J* = 7.5 Hz, 3H). **¹⁹F NMR (471 MHz, Chloroform-*d*) δ** -62.44. **¹³C NMR (126 MHz, Chloroform-*d*) δ** 185.12, 184.90, 150.21, 143.78, 142.81, 133.80, 133.75, 132.35, 132.05, 128.93 (q, *J =* 32.4 Hz), 128.89, 126.59, 126.50, 125.69 (q, *J* = 3.7 Hz), 124.29 (q, *J* = 271.9 Hz), 32.08, 21.08, 13.61.

### Example 36: Preparation of 2-phenyl-3-(4-(trifluoromethyl)benzyl) naphthalene-1,4-dione (MD603)(compound 3)

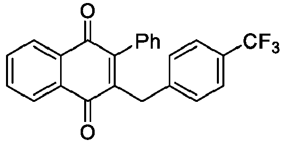

According to the general procedure, 2-phenylnaphthalene-1,4-dione and 4-trifluoromethylbenzyl bromide were used. After chromatography on silica gel (Cyclohexane:Toluene = 1:1, UV), 175 mg (0.45 mmol, 89% yield) of **MD603** were isolated as an orange solid. **¹H NMR (500 MHz, Chloroform-d) δ** 8.22 - 8.04 (m, 2H), 7.76 (m, 2H), 7.55-7.39 (m, 5H), 7.25-7.16 (m, 2H), 7.14 - 7.06 (m, 2H), 3.97 (s, 2H).**¹⁹F NMR (471 MHz, Chloroform-*d*) δ** - 62.44. **¹³C NMR (126 MHz, Chloroform-*d*) δ** 185.32,184.63,147.74,145.05,142.76, 134.13, 133.98, 133.22, 132.14, 129.22, 129.13, 129.04, 128.79 (q, *J* = 32.4 Hz), 128.55, 126.85, 126.67, 125.46 (q, *J* = 3.8 Hz), 124.29 (q, *J* = 271.8 Hz), 33.40.

### Example 37: Preparation of 1,4-dioxo-3-(4-(trifluoromethyl)benzyl)-1,4-dihydronaphthalen-2-yl acetate (MD611)

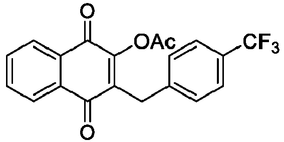

According to the general procedure, 1,4-dioxo-1,4-dihydronaphthalen-2-yl acetate and 4-trifluoromethylbenzyl bromide were used. After chromatography on silica gel (Cyclohexane:Toluene = 1:9, UV), 91 mg (0.24 mmol, 49% yield) of **MD611** were isolated as an orange solid. **¹H NMR (500 MHz, Chloroform-*d*) δ** 8.16 - 8.04 (m, 2H), 7.77 - 7.70 (m, 2H), 7.53 (d, *J* = 8.1 Hz, 2H), 7.41 (d, *J* = 8.4 Hz, 2H), 3.97 (s, 2H), 2.41 (s, 3H). **¹⁹F NMR (471 MHz, Chloroform-*d*) δ** -62.49. **¹³C NMR (126 MHz, Chloroform-*d*) δ** 184.23, 178.17, 167.91, 151.94, 141.44, 136.75, 134.47, 134.22, 131.91, 130.93, 129.41, 129.20 (q, *J* = 32.4 Hz), 127.01, 126.88, 125.71 (q, *J* = 3.8 Hz), 124.23 (q, *J* = 272.0 Hz), 30.12, 20.52.

### Example 38: Preparation of 2-phenoxy-3-(4-(trifluoromethyl)benzyl) naphthalene-1,4-dione (MD607)(compound 4)

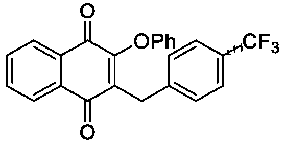

According to the general procedure, 2-phenoxynaphthalene-1,4-dione and 4-trifluoromethylbenzyl bromide were used. After chromatography on silica gel (Cyclohexane:Toluene = 1:1, UV), 85 mg (0.21 mmol, 42% yield) of **MD607** were isolated as a yellow solid. **¹H NMR (500 MHz, Chloroform-*d*) δ** 8.22 - 8.11 (m, 1H), 8.02 - 7.94 (m, 1H), 7.75 (td, *J* = 7.5, 1.5 Hz, 1H), 7.71 (td, *J* = 7.5, 1.5 Hz, 1H), 7.52 - 7.43 (m, 4H), 7.35 - 7.29 (m, 2H), 7.15 - 7.08 (m, 1H), 6.94 - 6.88 (m, 2H), 4.08 (s, 2H). **¹⁹F NMR (471 MHz, Chloroform-*d*) δ** -62.44. **¹³C NMR (126 MHz, Chloroform-*d*) δ** 184.89, 179.76, 157.14, 153.96, 142.20, 136.68, 134.39, 134.05, 131.97, 131.31, 129.90, 129.85, 128.99 (q, *J* = 32.4 Hz), 126.83, 126.77, 125.57 (q, *J* = 3.8 Hz), 124.30 (q, *J* = 272.0 Hz), 123.70, 116.36, 29.77.

### Example 39: Preparation of 2-benzyl-3-(4-(trifluoromethyl)benzyl) naphthalene-1,4-dione (MD614)(compound 5)

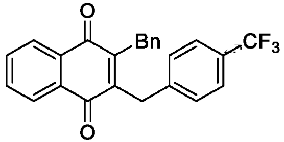

According to the general procedure, 2-benzylnaphthalene-1,4-dione and 4-trifluoromethylbenzyl bromide were used. After chromatography on silica gel (Cyclohexane:Toluene = 4:6, UV), 113 mg (0.28 mmol, 56% yield) of **MD614** were isolated as a yellow solid. **¹H NMR (500 MHz, Chloroform-*d*)** 8.17 - 8.01 (m, 2H), 7.83 - 7.66 (m, 2H), 7.48 (d, *J* = 8.1 Hz, 2H), 7.27 - 7.12 (m, 7H), 4.13 (s, 2H), 4.10 (s, 2H). **¹⁹F NMR (471 MHz, Chloroform-*d*) δ** -62.45. **¹³C NMR (126 MHz, Chloroform-*d*) δ** 185.15, 185.09, 146.78, 145.46, 142.18, 137.80, 133.97, 133.93, 132.13, 132.06, 128.97, 128.96 (q, *J* = 32.6 Hz), 128.90, 128.60, 126.78, 126.67, 125.66 (q, *J* = 3.9 Hz), 124.26 (q, *J* = 272.1 Hz), 32.63, 32.61.

### Example 40: Preparation of 2-(3-fluorobenzyl)-3-methylnaphthalene-1,4-dione (MRO0039)

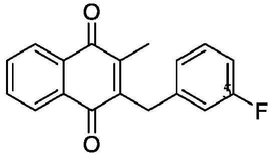

According to the general procedure, 2-benzylnaphthalene-1,4-dione and 3-fluorobenzyl bromide were used. After chromatography on silica gel (Cyclohexane:Toluene = 1:4, UV), 84 mg (0.30 mmol, 60% yield) of **MRO0039** were isolated as a yellow solid. **¹H NMR (400 MHz, Chloroform-*d*)** 7.96 (dd, *J* = 5.7, 3.3 Hz, 2H), 7.57 (dd, *J* = 5.8, 3.3 Hz, 2H), 7.15-7.07 (m, 1H), 6.92-6.87 (m, 1H), 6.83-6.72 (m, 2H), 3.90 (s, 2H), 2.12 (s, 3H). **¹⁹F NMR (377 MHz, Chloroform-*d*) δ** -112.88 (ddd, *J* = 9.9, 8.7, 6.0 Hz). **¹³C NMR (101 MHz, Chloroform-d) δ** 185.3, 184.6, 163.0 (d, *J* = 247.5 Hz), 144.8, 144.7, 140.6 (d, *J* = 8.1 Hz), 133.7 (2C), 132.2, 132.0, 130.1 (d, *J* = 9.1 Hz), 136.6, 126.4, 124.3 (d, *J* = 3.0 Hz), 115.6 (d, *J* = 21.2 Hz), 113.5 (d, *J* = 21.2 Hz), 32.2 (d, *J* = 2.0 Hz), 13.4.

### Example 41: Preparation of 2-(3-cyanobenzyl)-3-methylnaphthalene-1,4-dione (MRO0041)

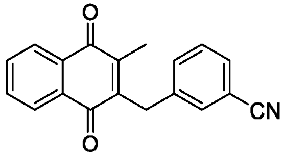

According to the general procedure, 2-benzylnaphthalene-1,4-dione and 3-cyanobenzyl bromide were used. After chromatography on silica gel (Cyclohexane:Toluene = 1:4, UV), 95 mg (0.33 mmol, 66% yield) of **MRO0041** were isolated as a yellow solid. **¹H NMR (400 MHz, Chloroform-*d*)** 8.09-8.01 (m, 2H), 7.72-7.66 (m, 2H), 7.52 7.45 (m, 3H), 7.36 (t, J = 7.7 Hz, 1H), 4.03 (s, 2H), 2.23 (s, 3H). **¹³C NMR (101 MHz, Chloroform-*d*) δ** 185.0, 184.4, 145.0, 143.9, 139.7, 133.8, 133.7, 133.2, 132.10, 132.07, 131.8, 130.3, 129.5, 126.6, 126.5, 118.7, 112.8, 32.1, 13.4.

### Example 42: Preparation of 3-benzyl-6-fluoro-2-methylnaphthalene-1,4-dione (MRO0010)

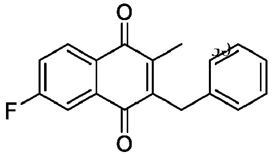

According to the general procedure, 6-fluoro-2-methylnaphthalene-1,4-dione and benzyl bromide were used. After chromatography on silica gel (Cyclohexane:Toluene = 3:7, UV), 94 mg (0.34 mmol, 67% yield) of **MRO0010** were isolated as a yellow solid. **¹H NMR (400 MHz, Chloroform-*d*)** 8.12 (dd, *J* = 8.6, 5.3 Hz, 1H), 7.72 (dd, *J* = 8.6, 2.7 Hz, 1H), 7.35 (td, *J* = 8.3, 2.7 Hz, 1H), 7.30-7.16 (m, 5H), 4.02 (s, 2H), 2.25 (s, 3H). **¹⁹F NMR (377 MHz, Chloroform-*d*) δ** -102.57 (td, *J* = 8.6, 5.3 Hz). **¹³C NMR (101 MHz, Chloroform-*d*) δ** 184.2, 183.6 (d, *J* = 1.0 Hz), 166.1 (d, *J* = 263.0 Hz), 145.6 (d, *J* = 2.0 Hz), 144.8, 137.9, 134.7 (d, *J* = 11.1 Hz), 129.7 (d, *J* = 9.1 Hz), 128.9, 128.8 (2C), 128.7 (2C), 126.7, 120.7 (d, *J* = 22.2 Hz), 113.3 (d, *J* = 23.2 Hz), 32.6, 13.4.

### Example 43: 3-((7-fluoro-3-methyl-1,4-dioxo-1,4-dihydronaphthalen-2-yl)methyl)benzonitrile (MRO0042)

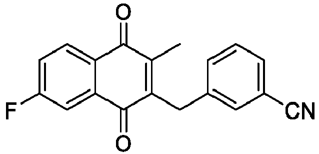

According to the general procedure, 6-fluoro-2-methylnaphthalene-1,4-dione and 3-cyanobenzyl bromide were used. After chromatography on silica gel (Cyclohexane:Toluene = 1:4, UV), 65 mg (0.34 mmol, 43% yield) of **MRO0042** were isolated as a yellow solid. **¹H NMR (400 MHz, Chloroform-*d*)** 8.12 (dd, *J* = 8.6, 5.2 Hz, 1H), 7.69 (dd, *J* = 8.5, 2.6 Hz, 1H), 7.51-7.45 (m, 3H), 7.41-7.33 (m, 2H), 4.04 (s, 2H), 2.24 (s, 3H). **¹⁹F NMR (377 MHz, Chloroform-*d*) δ** -102.04 (td, *J* = 8.3, 5.3 Hz). **¹³C NMR (101 MHz, Chloroform-*d*) δ** 183.7, 183.4 (d, *J* = 1.0 Hz), 166.1 (d, *J* = 258.6 Hz), 145.4, 144.1 (d, *J* = 2.0 Hz), 139.5, 134.4 (d, *J* = 8.1 Hz), 133.2, 132.1, 130.5, 129.8 (d, *J* = 9.1 Hz), 129.6, 128.7 (d, *J* = 4.0 Hz), 121.1 (d, *J* = 22.2 Hz), 118.7, 113.3 (d, *J* = 24.2 Hz), 112.9, 32.2, 13.5.

The process of the invention was also carried out with other reaction conditions, as shown hereafter.

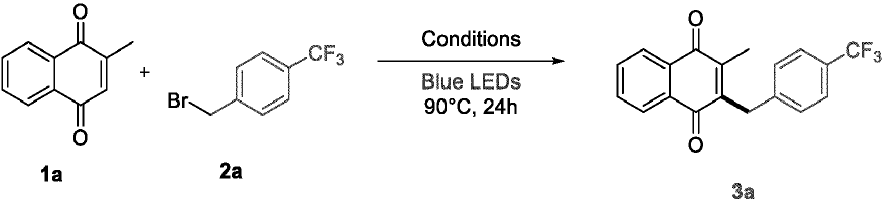

### Procedure:

In a 10 mL tube, quinone (0.5 mmol), benzyl bromide (0.75 mmol) and iron catalyst were dissolved in the solvent (5 mL). The mixture was put under agitation and base and HAT agent were added successively. The tube was sealed, put under blue light irradiation and heated up at 90°C during 24h. After completion, the mixture was allowed to cool down at room temperature and was partitioned between ethyl acetate (10 mL) and aqueous 1M HCl (10 mL). The aqueous layer was extracted once with 10 mL ethyl acetate and the reunited organic layers were washed with brine and dried with MgSO₄. The solvent was removed and the crude was purified by silica gel chromatography to obtained pure benzylated quinone.

| Solvent | Temperature (°C) | Cat (mol%) | Base (Equiv) | Reductant (Equiv) | Duration (h) | Yield 3a (%) |
|---|---|---|---|---|---|---|
| MeCN | 90°C | Fe(acac)₃ (10%) | 2,6-lutidine (1.2) | γ-terpinene (1.2) | 24h | 84% |
| MeCN | 90°C | Fe(acac)₃ (10%) | collidine (1.2) | γ-terpinene (1.2) | 24h | 73% |
| MeCN | 90°C | Fe(acac)₃ (10%) | DIPEA (1.2) | γ-terpinene (1.2) | 24h | 31% |
| MeCN | 90°C | Fe(acac)₃ (10%) | 2,6-lutidine (1.2) | Hantschz ester (1.2) | 24h | 72% |
| MeCN | 90°C | FeCl₃.6H₂O (10%) | 2,6-lutidine (1.2) | γ-terpinene (1.2) | 24h | 70% |
| MeCN | 90°C | FeCl₂ (10%) | 2,6-lutidine (1.2) | γ-terpinene (1.2) | 24h | 67% |
| MeCN | 90°C | Fe(acac)₃ (10%) | 2,6-lutidine (1.2) | γ-terpinene (0.5) | 24h | 71% |
| MeCN | 90°C | Fe(acac)₃ (20%) | 2,6-lutidine (1.2) | γ-terpinene (1.2) | 24h | 49% |
| MeCN | 90°C | Fe(acac)₃ (2%) | 2,6-lutidine (1.2) | γ-terpinene (1.2) | 24h | 35% |
| Toluene | 90°C | Fe(acac)₃ (10%) | 2,6-lutidine (1.2) | γ-terpinene (1.2) | 24h | 36% |
| Dichloroethane | 90°C | Fe(acac)₃ (10%) | 2,6-lutidine (1.2) | γ-terpinene (1.2) | 24h | 59% |
| Dimethylformamide | 90°C | Fe(acac)₃ (10%) | 2,6-lutidine (1.2) | γ-terpinene (1.2) | 24h | 35% |
| MeCN | 70°C | Fe(acac)₃ (10%) | 2,6-lutidine (1.2) | γ-terpinene (1.2) | 24h | 20% |
| MeCN | 100°C | Fe(acac)₃ (10%) | 2,6-lutidine (1.2) | γ-terpinene (1.2) | 24h | 79% |
| MeCN | 90°C | Fe(acac)₃ (10%) | 2,6-lutidine (1.2) | γ-terpinene (1.2) | 48h | 84% |

## Claims

1. A method for the preparation of a compound having the formula (I): 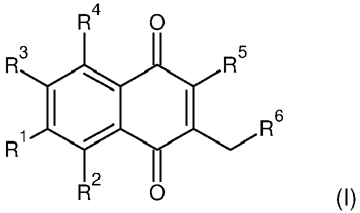 wherein:
• R¹, R², R³, and R⁴ are independently from each other selected from the group consisting of:
□ H,
□ halogen,
□ OH,
□ (C₁-C₆)alkyl,
□ -O(P=O)OAlk₂, Alk being a (C₁-C₆)alkyl group,
□ halo(C₁-C₆)alkyl,
□ halo(C₁-C₆)alkoxy, and
□ (C₁-C₆)alkoxy,
• R⁵ is selected from the group consisting of:
□ (C₁-C₆)alkyl,
□ OH,
□ halogen,
□ (C₆-C₁₀)aryl,
□ (C₆-C₁₀)aryloxy,
□ (C₆-C₁₀)aryl(C₁-C₆)alkyl, and
□ -O-C(=O)-(C₁-C₆)alkyl,
• R⁶ is a group of formula -CH=CH₂ or a group having the below formula (II): 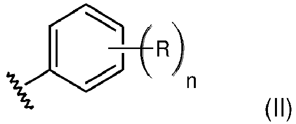 wherein:
□ n is 0 or an integer varying from 1 to 5; and
□ R, identical or different, is selected from the group consisting of:
. halo(C₁-C₆)alkyl,
. (C₁-C₆)alkyl,
. CN,
. NO₂,
. halogen,
. -C(=O)-(C₁-C₆)alkyl,
. halo(C₁-C₆)alkoxy,
. (C₁-C₆)alkoxy,
or two adjacent R groups may form together with the carbon atoms carrying them a (C₆-C₁₀)aryl group or a heterocycloalkyl group;
said method comprising a step of reacting a compound having the following formula (III): 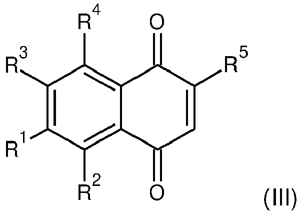
R¹, R², R³, R⁴, and R⁵ being as defined above in formula (I);
with a compound having the following formula (IV):
X-CH₂-R⁶ (IV)
R⁶ being as defined above in formula (I), and
X being a halogen atom;
said reacting step being carried out under light irradiation in the presence of an iron catalyst, a heteroatomic base, and a HAT agent.

2. The method of claim 1, wherein the reacting step is carried out at a temperature comprised from 80°C to 150°C, preferably at 90°C.

3. The method of claim 1 or 2, wherein the reacting step is carried out for a time comprised from 12 hours to 72 hours, preferably for 24 hours.

4. The method of any one of claims 1 to 3, wherein the iron catalyst is a Fe(II) or Fe(III) catalyst, in particular selected from the group consisting of: FeCl₂, FeCl₃, Fe(acac)₂, Fe(acac)₃, Fe(ClO₄)₃, Fe(NO₃)₃, ((NH₄)₂Fe(SO₄)₂·6H₂O).

5. The method of any one of claims 1 to 4, wherein the heteroatomic base is selected from the group consisting of: pyridine, picoline, 2,6-lutidine, collidine, and DIPEA, and is preferably 2,6-lutidine.

6. The method of any one of claims 1 to 5, wherein the HAT agent is selected from the group consisting of: Hantzsch's esters, analogues thereof, and γ-terpinene.

7. The method of any one of claims 1 to 6, wherein the reacting step is carried out in a solvent, said solvent being in particular selected from the group consisting of: acetonitrile, toluene, hexafluoroisopropanol, dichloroethane, dimethylformamide, ethyl acetate, and isopropanol, and is preferably acetonitrile.

8. The method of any one of claims 1 to 7, wherein the reacting step is carried out under blue light irradiation.

9. The method of any one of claims 1 to 8, wherein the amount of catalyst is comprised between 2% and 20% in moles in relation to the number of moles of compound of formula (III).

10. The method of any one of claims 1 to 9, wherein the amount of heteroatomic base is comprised from 1 to 5 equivalents.

11. The method of any one of claims 1 to 10, wherein the amount of HAT agent is comprised from 0.2 to 5 equivalents.

12. The method of any one of claims 1 to 11, wherein the amount of compound of formula (IV) is comprised from 1 to 5 equivalents, and is preferably equal to 1.5 equivalent, in relation to the amount of compound of formula (III).

13. The method of any one of claims 1 to 12, wherein, in formula (I):
• R¹ is selected from the group consisting of: H, halogen, -O(P=O)OAlk₂, Alk being as defined in claim 1, hal0(C₁-C₆)alkyl, halo(C₁-C₆)alkoxy, and (C₁-C₆)alkoxy;
• R² is selected from the group consisting of: H, OH, and (C₁-C₆)alkoxy; and
• R³ and R⁴ are H.

14. The method of claim 13, wherein R¹ is H or F, and R² is selected from the group consisting of: H, OH, and (C₁-C₆)alkoxy.

15. A compound having the formula (V): 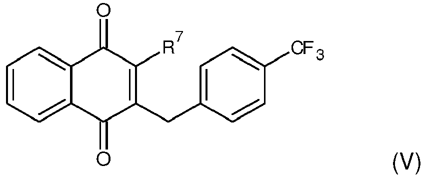 R⁷ being selected from the group consisting of:
□ halogen, such as Cl or Br,
□ (C₆-C₁₀)aryl, such as Ph,
□ (C₆-C₁₀)aryloxy, such as OPh, and
□ (C₆-C₁₀)aryl(C₁-C₆)alkyl, such as benzyl,
said compound of formula (V) being preferably selected from the following compounds:
